(19) **Europäisches Patentamt**
**European Patent Office**
**Office européen des brevets**

(11) **EP 2 821 501 B1**

(12) **EUROPEAN PATENT SPECIFICATION**

(45) Date of publication and mention
of the grant of the patent:
**19.09.2018 Bulletin 2018/38**

(51) Int Cl.:
***C12Q 1/68*** *(2018.01)*

(21) Application number: **12870229.7**

(86) International application number:
**PCT/CN2012/071648**

(22) Date of filing: **27.02.2012**

(87) International publication number:
**WO 2013/127049 (06.09.2013 Gazette 2013/36)**

(54) **METHOD AND DEVICE FOR DETECTING MICRODELETION IN CHROMOSOME STS AREA**

VERFAHREN UND VORRICHTUNG ZUR ERKENNUNG VON MIKRODELETION IN EINEM
CHROMOSOMALEN STS-BEREICH

MÉTHODE ET DISPOSITIF DE DÉTECTION D'UNE MICRODÉLÉTION DANS UNE RÉGION DE STS
DE CHROMOSOME

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**07.01.2015 Bulletin 2015/02**

(73) Proprietor: **BGI Genomics Co., Ltd.**
**Yantian District**
**Guangdong Province 518083 (CN)**

(72) Inventors:
• **LIU, Xiao**
**Shenzhen**
**Guangdong 518083 (CN)**
• **ZHANG, Junjie**
**Shenzhen**
**Guangdong 518083 (CN)**
• **XU, Huaiqian**
**Shenzhen**
**Guangdong 518083 (CN)**
• **SU, Zheng**
**Shenzhen**
**Guangdong 518083 (CN)**
• **ZHANG, Ruifang**
**Shenzhen**
**Guangdong 518083 (CN)**
• **WANG, Jun**
**Shenzhen**
**Guangdong 518083 (CN)**
• **WANG, Jian**
**Shenzhen**
**Guangdong 518083 (CN)**
• **YANG, Huanming**
**Shenzhen**
**Guangdong 518083 (CN)**

(74) Representative: **Rössler, Matthias et al**
**Kahlhöfer Rößler Kreuels**
**Patentanwälte PartG mbB**
**(Karo IP Professionals)**
**Platz der Ideen 2**
**40476 Düsseldorf (DE)**

(56) References cited:
**WO-A1-2008/030065      KR-A- 20090 112 235**
**US-A1- 2009 176 226      US-A1- 2009 203 014**

• **GROSS SUSAN J ET AL: "Rapid and novel
prenatal molecular assay for detecting
aneuploidies and microdeletion syndromes.",
PRENATAL DIAGNOSIS MAR 2011, vol. 31, no. 3,
March 2011 (2011-03), pages 259-266,
XP002745167, ISSN: 1097-0223**
• **QING WU ET AL: "Prevalent false positives of
azoospermia factor a (AZFa) microdeletions
caused by single-nucleotide polymorphism
rs72609647 in the sY84 screening of male
infertility", ASIAN JOURNAL OF ANDROLOGY,
vol. 13, no. 6, 18 July 2011 (2011-07-18), pages
877-880, XP055216707, ISSN: 1008-682X, DOI:
10.1038/aja.2011.51**
• **META-GENOMICS DNA SEQUENCING 20
December 2009, XP008174376 Retrieved from the
Internet: <URL:http://biolab. blogus. com/logs
54461787.html>**

Note: Within nine months of the publication of the mention of the grant of the European patent in the European Patent
Bulletin, any person may give notice to the European Patent Office of opposition to that patent, in accordance with the
Implementing Regulations. Notice of opposition shall not be deemed to have been filed until the opposition fee has been
paid. (Art. 99(1) European Patent Convention).

**(Cont. next page)**

• YEOM ET AL: "Application of multiplex bead array assay for Yq microdeletion analysis in infertile males", MOLECULAR AND CELLULAR PROBES, ACADEMIC PRESS, LONDON, GB, vol. 22, no. 2, 6 March 2008 (2008-03-06), pages 76-82, XP022513578, ISSN: 0890-8508, DOI: 10.1016/J.MCP.2007.06.004

**Description**

TECHNICAL FIELD

Embodiments of the present disclosure generally relate to genetic engineering technology, more particularly to a method of detecting a micro-deletion in a sequence tagged site region of a chromosome and an apparatus thereof.

BACKGROUND

Deletion is a phenomenon of occurring partial loss of chromosome or DNA molecules in a genome, which is one most important reason leading to genetic mutation.

Currently, PCR (Polymerase Chain Reaction) technique is normally used in detecting a micro-deletion in a sequence tagged site (STS) region of a chromosome. The PCR technique is a method of selectively amplify certain of special region in DNA in vitro by means of simulating a pattern of DNA replication in vivo. Using PCR technique has advantages of rapidity and convenience when detecting a small amount of sites; besides it requires knowing the sequences at both ends of a certain region of DNA in advance for probe design, by which it requires that these micro-deletions in the sequence tagged site region of the chromosome should be reported in advance.

KR 2009 0112235 A discloses a screening test of idiopathic infertility wherein an analysis chip of human Y chromosome microdeletion is provided to quickly analyze 19 kinds of STS marker and five genes for detecting microdelection of AZFa, AZFb, and AZFc sites. The screening test comprised: a step of designing a chip in which 25 kinds of probes are spotted; a step of isolating genomic DNA through magnetic bead from blood or semen; a step of amplifying nine kinds of AZFa, ten kinds of AZFb, AND nine kinds of AZFc STS marker or gene through PCR; a step of inducing hybridization between the probes; and a step of scanning the chip and measuring fluorosecence intensity.

But, when facing a large amount of samples or unreported micro-deletions, detection using PCR technique may have a huge limitation; in addition, when the sequence tagged site region to be detected having a relative larger number of sites, or particularly when detecting, the traditional detection using PCR technique has been unable to meet such requirement, thus a novel method for research is further required.

SUMMARY

A main technical problem solved by the present disclosure is to provide a method of detecting a micro-deletion in a sequence tagged site region of a chromosome and an apparatus thereof, which may detect the micro-deletion in the sequence tagged site region of the chromosome when facing a large amount of samples with limited cost, and may also detect unreported micro-deletions in the sequence tagged site region of the chromosome.

To solve the above technical problem, one technical solution used by the present disclosure is: providing a method of detecting a micro-deletion in a sequence tagged site region of a chromosome, which comprises:

obtaining a capture probe corresponding to DNA sequence of the sequence tagged site region;
hybridizing the capture probe with a library of a plurality of DNA samples, to capture the DNA sequence of the sequence tagged site region from the plurality of DNA samples;
sequencing the captured DNA sequence of the sequence tagged site region to obtain sequencing data; and
analyzing the sequencing data using a mathematical statistics method, to obtain a result showing a presence or an absence of the micro-deletion in the sequence tagged site region of the chromosome in each of the plurality of DNA samples.

Preferably, the step of analyzing the sequencing data using the mathematical statistics test further comprises:

subjecting a sequencing depth value of the sequence tagged site region to normalization, to obtain a normalized sequencing depth value; and
detecting an outlier of the sequencing depth value of the sequence tagged site region based on the normalized sequencing depth value using the mathematical statistics method, to obtain the result showing the presence or the absence of the micro-deletion in the sequence tagged site region of the chromosome in each of the plurality of DNA samples.

Preferably, the step of subjecting the sequencing depth value of the sequence tagged site region to normalization further comprises:
dividing all of the sequencing depth values of each of the plurality of DNA samples for a same region by an average depth value of each of the plurality of DNA samples, to obtain the normalized depth values of the plurality of DNA samples for the same region.

Preferably, the step of detecting the outlier of the sequencing depth value of the sequence tagged site region further comprises:

calculating a mean value and a variance based on all normalized sequencing depth values of each of the plurality of DNA samples for a same region;

obtaining a normal distribution curve with all of non-outliers for the same region, based on the calculated mean value and variance of all normalized depth values of each of the plurality of DNA samples for the same region;

calculating a probability for each of the plurality of DNA samples in every region with a specific sequencing depth value, based on the normal distribution curve;

determining a first probability cut-off value based on the probability for each of the plurality of DNA samples in a given region with the specific sequencing depth value; and

obtaining a result R1 showing the presence of the micro-deletion in the given region, if the probability for one of the plurality of DNA samples in the given region with the specific sequencing depth value is smaller than the first probability cut-off value.

Preferably, wherein after the step of obtaining the result R1, the method further comprises:

subjecting the result R1 to experimental verification, to obtain a verification result;

determining a second probability cut-off value based on the verification result, wherein the second probability cut-off value is smaller than the first probability cut-off value; and

obtaining a result R2 showing the presence of the micro-deletion in the given region, if the probability for one of the plurality of DNA samples in the given region with the specific depth value is smaller than the second probability cut-off value.

Preferably, wherein the step of detecting the outlier of the sequencing depth value of the sequence tagged site region further comprises:

calculating a first ratio D/S between the normalized depth value of the plurality of DNA samples for a given region and a median of all sequencing depth values of each of the plurality of DNA samples, based on the normalized depth value of the sequence tagged site region of the plurality of the plurality of DNA samples;

calculating a first ratio D/R between the normalized depth value of the plurality of DNA samples for the given region and a median of the sequencing depth values of all regions, based on the normalized depth value of the sequence tagged site region of the plurality of DNA samples;

training a first ratio cut-off value with the first ratio D/S using ID3 algorithm, and training a second ratio cut-off value with the first ratio D/R using ID3 algorithm;

obtaining the result showing the absence of the micro-deletion in the given region of the plurality of DNA samples, if the first ratio D/S is greater than the first ratio cut-off value;

obtaining the result showing the absence of the micro-deletion in the given region of the plurality of DNA samples, if the first ratio D/S is smaller than the first ratio cut-off value and the first ratio D/R is greater than the second ratio cut-off value; and

obtaining the result showing the presence of the micro-deletion in the given region of the plurality of DNA samples, if the first ratio D/S is smaller than the first ratio cut-off value and the first ratio D/R is smaller than the second ratio cut-off value.

Preferably, the step of detecting the outlier of the sequencing depth value of the sequence tagged site region further comprises:

calculating a mean value and a variance based on all normalized depth values of each of the plurality of DNA samples for a same region;

obtaining a normal distribution curve with all of non-outliers for the same region, based on the calculated mean value and variance of all normalized depth values of each of the plurality of DNA samples for the same region;

calculating a probability for each of the plurality of DNA samples in every region with a specific sequencing depth value, based on the normal distribution curve;

determining a third probability cut-off value based on the probability for each of the plurality of DNA samples in a given region with the specific sequencing depth value;

obtaining a result R3 showing the presence of the micro-deletion in the given region, if the probability for one of the plurality of DNA samples in the given region with the specific sequencing depth value is smaller than the third probability cut-off value;

calculating a second ratio D/S between the normalized depth value of the plurality of DNA samples for the given region and a median of all sequencing depth values of each of the plurality of DNA samples in the result R3;

calculating a second ratio D/R between the normalized depth value of the plurality of DNA samples for the given region and a median of the sequencing depth values of all regions in the result R3;

training a third ratio cut-off value with the second ratio D/S using ID3 algorithm, and training a fourth ratio cut-off value with the second ratio D/R using ID3 algorithm;

obtaining the result showing the absence of the micro-deletion in the given region of the plurality of DNA samples, if the second ratio D/S is greater than the third ratio cut-off value;

obtaining the result showing the absence of the micro-deletion in the given region of the plurality of DNA samples, if the second ratio D/S is smaller than the third ratio cut-off value and the second ratio D/R is greater than the fourth ratio cut-off value; and

obtaining the result showing the presence of the micro-deletion in the given region of the plurality of DNA samples, if the second ratio D/S is smaller than the third ratio cut-off value and the second ratio D/R in the result R3 is smaller than the fourth ratio cut-off value.

Preferably, the step of obtaining the capture probe further comprises:

finding the DNA sequence of the sequence tagged site region within a genomic database;

selecting a qualified DNA sequence of the sequence tagged site region meeting requirement for designing the capture probe; and

designing and synthesizing the capture probe based on the qualified DNA sequence.

Preferably, the library of the plurality of DNA samples is constructed by following steps:

constructing a plurality of DNA libraries for a plurality of single DNA samples, wherein each of the plurality of DNA libraries has a different adaptor respectively;

mixing the plurality of DNA libraries with a predetermined ratio; and

determining the resulting mixture as the mixture library, if the resulting mixture meets a requirement of quality control.

Preferably, in the step of constructing a plurality of DNA libraries for a plurality of single DNA samples, for each of the plurality of single DNA samples, the DNA library is constructed by following steps:

breaking a genome DNA into DNA fragments having a predetermined size by means of physical or chemical method, to obtain DNA fragments;

subjecting the DNA fragments to end-repairing, to obtain end-repaired DNA fragments with a phosphorylated end;

adding an "A" base to the end-repaired DNA fragments at 3'-end to obtain DNA fragments with the "A" base at 3'-end;

ligating an Index Adaptor to the DNA fragments with the "A" base at 3'-end, to obtain DNA fragments ligated with the Index Adaptor;

amplifying the DNA fragments ligated with the Index Adaptor using a primer comprising a sequence specific to the Index Adaptor, to obtain an amplification product; and

determining the amplification product as the DNA library, if the amplification product meets the requirement of quality control.

Preferably, after the step of sequencing the captured DNA sequencing and prior to the step of analyzing the sequencing data, the method further comprises:

subjecting the sequencing data to a quality control.

Preferably, the step of subjecting the sequencing data to the quality control further comprises:

filtering out first unqualified data in the sequencing data, to obtain first qualified sequencing data;

aligning the first qualified sequencing to a reference genome sequence by short-sequence alignment software, and calculating a first correlation parameter of the sequencing depth value of each of the plurality of DNA samples and a second correlation parameter of the sequencing depth value for a given sequence tagged site region among the plurality of DNA samples;

filtering out second unqualified sequencing data based on the first calculated correlation parameter, to obtain second qualified sequencing data; and

filtering out third unqualified sequencing data based on the second calculated correlation parameter, to obtain third qualified sequencing data of the given sequence tagged site region.

Preferably, the step of filtering out the first unqualified data in the sequencing data, to obtain the first qualified sequencing data further comprises:

obtaining a first sequencing data set by filtering out unqualified data of which contains more than a predetermined proportion of bases having a low-quality from the sequencing data;
obtaining a second sequencing data set by filtering out unqualified data of which contains more than 10% undetermined bases from the first sequencing data set;
obtaining a third sequencing data set by filtering out unqualified data of which contains a sequencing adaptor sequence from the second sequencing data set after being aligned to a sequencing adaptor sequence library; and
obtaining the first qualified sequencing data by filtering out unqualified data of which contains an exogenous sequence from the third sequencing data set after being aligned to all introduced exogenous sequences.

Preferably, the step of filtering out the second unqualified sequencing data further comprises:

obtaining a lower quartile Q1, an upper quartile Q3 and an interquartile IQR by Quartile function and ranking all sequencing depth values of the plurality of DNA samples in the first qualified sequencing data in an ascending order; and
filtering out unqualified sequencing data of which has a sequencing depth value beyond a range of from Q1-1.5IQR to Q3+1.5IQR, to obtain the second qualified sequencing data.

Preferably, the step of filtering out the third unqualified sequencing data further comprises:

obtaining a lower quartile Q1, an upper quartile Q3 and an interquartile IQR by Quartile function and ranking all sequencing depth values of the given region among different DNA samples in the second qualified sequencing data in an ascending order; and
filtering out unqualified sequencing data of which a median of the sequencing depth values of the given region among different DNA samples is zero or greater than Q3+1.5IQR, to obtain the third qualified sequencing data.

As reference, another technical solution is described herewith: providing an apparatus for detecting a microdeletion in a sequence tagged site region of a chromosome, which may comprises:

a capture probe obtaining module, for obtaining a capture probe corresponding to DNA sequence of the sequence tagged site region;
a hybridizing module, for hybridizing the capture probe with a mixture library of a plurality of DNA samples, to capture the DNA sequence of the sequence tagged site region from the plurality of DNA samples;
a sequencing data obtaining module, for sequencing the captured DNA sequence of the sequence tagged site region to obtain sequencing data; and
a micro-deletion result obtaining module, for analyzing the sequencing data using a mathematical statistics method, to obtain a result showing a presence or an absence of the micro-deletion in the sequence tagged site region of the chromosome in each of the plurality of DNA samples.

Preferably, the micro-deletion result obtaining module further comprises:

a sequencing depth value normalizing unit, for subjecting a sequencing depth value of the sequence tagged site region to normalization, to obtain a normalized sequencing depth value; and
a micro-deletion result obtaining unit, for detecting an outlier of the sequencing depth value of the sequence tagged site region based on the normalized sequencing depth value using the mathematical statistics method, to obtain the result showing the presence or the absence of the micro-deletion in the sequence tagged site region of the chromosome in each of the plurality of DNA samples.

Preferably, the sequencing depth value normalizing unit is used in dividing all of the sequencing depth values of each of the plurality of DNA samples for a same region by an average depth value of each of the plurality of DNA samples, to obtain the normalized depth values of the plurality of DNA samples for the same region.
Preferably, the micro-deletion result obtaining unit further comprises:

a mean value and variance obtaining module, for calculating a mean value and a variance based on all normalized sequencing depth values of each of the plurality of DNA samples for a same region;
a normal distribution curve obtaining module, for obtaining a normal distribution curve with all of non-outliers for the same region, based on the calculated mean value and variance of all normalized depth values of each of the plurality of DNA samples for the same region;

a probability calculating module, for calculating a probability for each of the plurality of DNA samples in every region with a specific sequencing depth value, based on the normal distribution curve; and

a first determining module, for determining a first probability cut-off value based on the probability for each of the plurality of DNA samples in a given region with the specific sequencing depth value, and obtaining a result R1 showing the presence of the micro-deletion in the given region, if the probability for one of the plurality of DNA samples in the given region with the specific sequencing depth value is smaller than the first probability cut-off value.

Preferably, the micro-deletion obtaining unit still further comprises:

a probability cut-off value determining module, for subjecting the result R1 to experimental verification, to obtain a verification result, and determining a second probability cut-off value based on the verification result, wherein the second probability cut-off value is smaller than the first probability cut-off value; and

a second determining module, for obtaining a result R2 showing the presence of the micro-deletion in the given region, if the probability for one of the plurality of DNA samples in the given region with the specific depth value is smaller than the second probability cut-off value.

Preferably, the micro-deletion obtaining unit still further comprises:

a first ratio D/S obtaining module, for calculating a first ratio D/S between the normalized depth value of the plurality of DNA samples for a given region and a median of all sequencing depth values of each of the plurality of DNA samples, based on the normalized depth value of the sequence tagged site region of the plurality of the plurality of DNA samples;

a first ratio D/R obtaining module, for calculating a first ratio D/R between the normalized depth value of the plurality of DNA samples for the given region and a median of the sequencing depth values of all regions, based on the normalized depth value of the sequence tagged site region of the plurality of DNA samples;

a first ratio cut-off value obtaining module, for training a first ratio cut-off value with the first ratio D/S using ID3 algorithm, and training a second ratio cut-off value with the first ratio D/R using ID3 algorithm;

a third determining module, for obtaining the result showing the absence of the micro-deletion in the given region of the plurality of DNA samples, if the first ratio D/S is greater than the first ratio cut-off value;

a fourth determining module, for obtaining the result showing the absence of the micro-deletion in the given region of the plurality of DNA samples, if the first ratio D/S is smaller than the first ratio cut-off value and the first ratio D/R is greater than the second ratio cut-off value; and

a fifth determining module, for obtaining the result showing the presence of the micro-deletion in the given region of the plurality of DNA samples, if the first ratio D/S is smaller than the first ratio cut-off value and the first ratio D/R is smaller than the second ratio cut-off value.

Preferably, the micro-deletion obtaining unit still further comprises:

a mean value and variance obtaining module, for calculating a mean value and a variance based on all normalized sequencing depth values of each of the plurality of DNA samples for a same region;

a normal distribution curve obtaining module, for obtaining a normal distribution curve with all of non-outliers for the same region, based on the calculated mean value and variance of all normalized depth values of each of the plurality of DNA samples for the same region;

a probability calculating module, for calculating a probability for each of the plurality of DNA samples in every region with a specific sequencing depth value, based on the normal distribution curve;

a sixth determining module, for determining a third probability cut-off value based on the probability for each of the plurality of DNA samples in a given region with the specific sequencing depth value, and obtaining a result R3 showing the presence of the micro-deletion in the given region, if the probability for one of the plurality of DNA samples in the given region with the specific sequencing depth value is smaller than the third probability cut-off value;

a second ratio D/S obtaining module, for calculating a second ratio D/S between the normalized depth value of the plurality of DNA samples for the given region and a median of all sequencing depth values of each of the plurality of DNA samples in the result R3;

a second ratio D/R obtaining module, for calculating a second ratio D/R between the normalized depth value of the plurality of DNA samples for the given region and a median of the sequencing depth values of all regions in the result R3;

a second ratio cut-off value determining module, for training a third ratio cut-off value with the second ratio D/S using ID3 algorithm, and training a fourth ratio cut-off value with the second ratio D/R using ID3 algorithm;

a seventh determining module, for obtaining the result showing the absence of the micro-deletion in the given region of the plurality of DNA samples, if the second ratio D/S is greater than the third ratio cut-off value;

an eighth determining module, for obtaining the result showing the absence of the micro-deletion in the given region of the plurality of DNA samples, if the second ratio D/S is smaller than the third ratio cut-off value and the second ratio D/R is greater than the fourth ratio cut-off value; and

a ninth determining module, for obtaining the result showing the presence of the micro-deletion in the given region of the plurality of DNA samples, if the second ratio D/S is smaller than the third ratio cut-off value and the second ratio D/R in the result R3 is smaller than the fourth ratio cut-off value.

Preferably, the capture probe obtaining module further comprises:

a region finding unit, for finding the DNA sequence of the sequence tagged site region within a genomic database;

a sequence selecting unit, for selecting a qualified DNA sequence of the sequence tagged site region meeting requirement for designing the capture probe; and

a capture probe obtaining unit, for designing and synthesizing the capture probe based on the qualified DNA sequence.

Preferably, the apparatus described herewith may further comprise a mixture library constructing module, wherein the mixture library constructing module further comprises:

a plurality of DNA libraries constructing unit, for constructing a plurality of DNA libraries for a plurality of single DNA samples, wherein each of the plurality of DNA libraries has a different adaptor respectively;

a mixing unit, for mixing the plurality of DNA libraries with a predetermined ratio; and

a mixture library constructing unit, for determining the resulting mixture as the mixture library, if the resulting mixture meets a requirement of quality control.

Preferably, the apparatus described herewith may further comprise a quality control module, wherein the quality control module further comprises:

a first filtering unit, for filtering out first unqualified data in the sequencing data, to obtain first qualified sequencing data;

a sequencing depth value calculating unit, for aligning the first qualified sequencing to a reference genome sequence by short-sequence alignment software, and calculating a first correlation parameter of the sequencing depth value of each of the plurality of DNA samples and a second correlation parameter of the sequencing depth value for a given sequence tagged site region among the plurality of DNA samples;

a second filtering unit, for filtering out second unqualified sequencing data based on the first calculated correlation parameter, to obtain second qualified sequencing data; and

a third filtering unit, for filtering out third unqualified sequencing data based on the second calculated correlation parameter, to obtain third qualified sequencing data of the given sequence tagged site region.

To solve the above technical problem, a further technical solution used by the present disclosure is: providing computer a readable medium comprising an order, configured to perform the above method by a processer.

Advantages of the present disclosure may comprise: being different from the prior art, the method of detecting the micro-deletion in the sequence tagged site region of the chromosome and the apparatus thereof, by which obtains the capture probe covering an entire sequence tagged site region of a chromosome and captures DNA sequence of the sequence tagged site region from a plurality of DNA samples after being hybridized with a mixture library of the plurality of DNA samples, may efficiently and accurately detect reported or unreported micro-deletions in a sequence tagged site-related region of a chromosome with a large amount of DNA samples; besides the process of the analyzing the sequencing data using a mathematical statistics method is scientific, stable, with a high sensitivity, a low false positive rate, which may be used in effective analysis for the micro-deletions.

BRIEF DESCRIPTION OF THE DRAWINGS

Fig.1 is a flow chart showing a method of detecting a micro-deletion in a sequence tagged site region of a chromosome according to an embodiment of the present disclosure;

Fig.2 is a flow chart showing a method of detecting a micro-deletion in a sequence tagged site region of a chromosome according to another embodiment of the present disclosure;

Fig.3 is a flow chart showing a method of detecting a micro-deletion in a sequence tagged site region of a chromosome according to a further embodiment of the present disclosure;

Fig.4 is a flow chart showing a method of detecting a micro-deletion in a sequence tagged site region of a chromosome according to still another embodiment of the present disclosure;

Fig.5 is a flow chart showing a method of detecting a micro-deletion in a sequence tagged site region of a chromosome according to yet another embodiment of the present disclosure;

Fig.6 is a flow chart showing a method of detecting a micro-deletion in a sequence tagged site region of a chromosome according to still another embodiment of the present disclosure;

Fig.7 is a flow chart showing a method of detecting a micro-deletion in a sequence tagged site region of a chromosome according to yet another embodiment of the present disclosure;

Fig.8 is a histogram showing a filtered out sample having an outlier of sequencing depth value by means of a method of detecting a micro-deletion in a sequence tagged site region of a chromosome according to an embodiment of the present disclosure;

Fig.9 is a histogram showing a sample having normal sequencing depth value by means of a method of detecting a micro-dleletion in a sequence tagged site region of a chromosome according to an embodiment of the present disclosure;

Fig.10 is a flow chart showing a method of detecting a micro-deletion in a sequence tagged site region of a chromosome according to another embodiment of the present disclosure;

Fig.11 is a histogram showing a filtered out region having an outlier of sequencing depth value by means of a method of detecting a micro-deletion in a sequence tagged site region of a chromosome according to an embodiment of the present disclosure;

Fig.12 is a flow chart showing a method of detecting a micro-deletion in a sequence tagged site region of a chromosome according to a further embodiment of the present disclosure;

Fig.13 is a flow chart showing a method of detecting a micro-deletion in on a sequence tagged site region of a chromosome according to still another embodiment of the present disclosure;

Fig.14 is a flow chart showing a method of detecting a micro-deletion in on a sequence tagged site region of a chromosome according to yet another embodiment of the present disclosure;

Fig.15 is a flow chart showing a method of detecting a micro-deletion in a sequence tagged site region of a chromosome according to still another embodiment of the present disclosure;

Fig.16 is a diagram showing an example illustrating a decision-making tree constructed by ID3 algorithm in a method of detecting a micro-deletion in a sequence tagged site region of a chromosome according to an embodiment of the present disclosure;

Fig.17 is a partial flow chart showing decision-making tree analysis in a method of detecting a micro-deletion in a sequence tagged site region of a chromosome according to an embodiment of the present disclosure;

Fig.18 is a flow chart showing decision-making tree analysis in a method of detecting a micro-deletion in on a sequence tagged site region of a chromosome according to another embodiment of the present disclosure;

Fig.19 is a flow chart showing a method of detecting a micro-deletion in a sequence tagged site region of a chromosome according to a further embodiment of the present disclosure;

Fig.20 comprises a histogram and a box chart showing a median of sequencing depth value of a sample in a method of detecting a micro-deletion in a sequence tagged site region of a chromosome according to an embodiment of the present disclosure;

Fig.21 comprises a histogram and a box chart showing a median of sequencing depth value of a region by a method of detecting a micro-deletion in a sequence tagged site region of a chromosome according to an embodiment of the present disclosure;

Fig.22 is a diagram showing a probability when a sequencing depth value is a certain value in a method of detecting a micro-deletion in a sequence tagged site region of a chromosome according to an embodiment of the present disclosure;

Fig.23 is a diagram showing a probability threshold and a relationship between an expectation of the probability threshold and an observation of the probability threshold in a method of detecting a micro-deletion in a sequence tagged site region of a chromosome according to an embodiment of the present disclosure;

Fig.24 is a diagram showing a ratio of a true positive rate to a false positive rate in a method of detecting a micro-deletion in a sequence tagged site region of a chromosome according to an embodiment of the present disclosure;

Fig. 25 is a schematic diagram showing an apparatus for detecting a micro-deletion in a sequence tagged site region of a chromosome according to an embodiment of the present disclosure;

Fig.26 is a schematic diagram showing an apparatus for detecting a micro-deletion in a sequence tagged site region of a chromosome according to another embodiment of the present disclosure;

Fig.27 is a schematic diagram showing an apparatus for detecting a micro-deletion in a sequence tagged site region of a chromosome according to a further embodiment of the present disclosure;

Fig.28 is a schematic diagram showing an apparatus for detecting a micro-deletion in a sequence tagged site region of a chromosome according to still another embodiment of the present disclosure;

Fig.29 is a schematic diagram showing an apparatus for detecting a micro-deletion in a sequence tagged site region of a chromosome according to yet another embodiment of the present disclosure.

## DETAILED DESCRIPTION

Reference will be made in detail to drawings and embodiments of the present disclosure. In addition, terms such as "first" and "second" are used herein for purposes of description and are not intended to indicate or imply relative importance or significance.

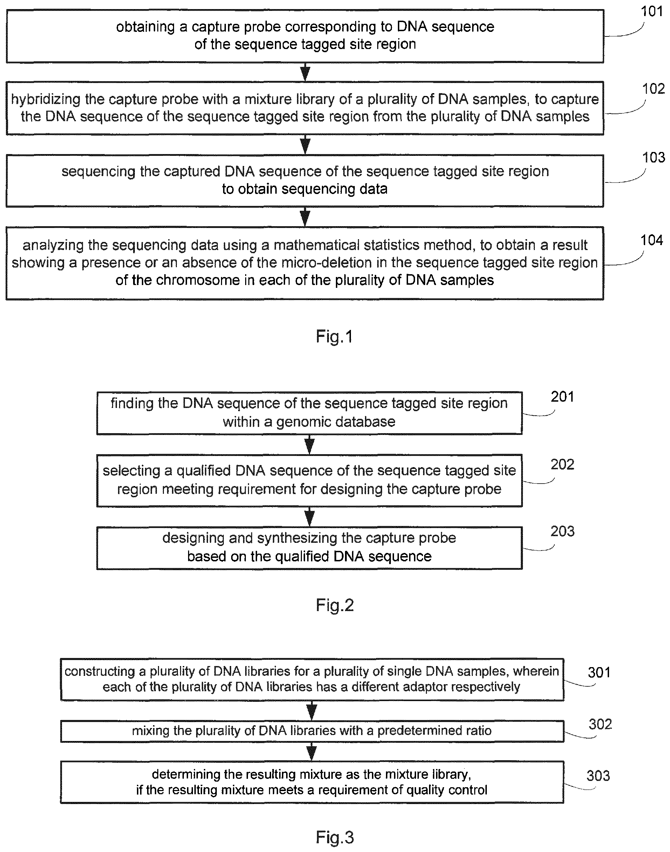

Fig.1 is a flow chart showing a method of detecting a micro-deletion in a sequence tagged site region of a chromosome according to an embodiment of the present disclosure. As shown in Fig.1, the method comprises:

step 101: obtaining a capture probe corresponding to DNA sequence of the sequence tagged site region.

Sequence tagged-site (STS) is a short single-copy DNA sequence of known map location serving as a marker, which can be uniquely-mapped by PCR amplification, for producing a site for map-making, namely, detection with an order of a series of STS may make a map of a genomic region. A probe is a short single-strand DNA or RNA fragment (about 20 to 500 bp), for detecting a complementary nucleic acid sequence thereof.

step 102: hybridizing the capture probe with a mixture library of a plurality of DNA samples, to capture the DNA sequence of the sequence tagged site region from the plurality of DNA samples.

The expression "mixture libraries" used herein refers to all DNA sequences for sequencing obtained by mixing a plurality of DNA libraries for a plurality of single DNA samples. Specific steps for constructing the mixture library are described below in details.

step 103: sequencing the captured DNA sequence of the sequence tagged site region to obtain sequencing data.

step 104: analyzing the sequencing data using a mathematical statistics method, to obtain a result showing a presence or an absence of the micro-deletion in the sequence tagged site region of the chromosome in each of the plurality of DNA samples.

Mathematical statistics is one branch of mathematics developing with a development of probability theory, studying on how to effectively optimize, arrange and analyze data affected by random factors, by which may infer or predict an issue under consideration, providing basis and suggestions for making a certain decision or taking a certain action.

In an embodiment, as shown in Fig.2, the step of obtaining the capture probe further comprises:

step 201: finding the DNA sequence of the sequence tagged site region within a genomic database.

Within a sequence tagged site region of chromosome Y in UCSC database, at a location coordinate of human genome reference sequence Hg19 (http://genome.ucsc.edu/), DNA sequence of the sequence tagged site region in chromosome Y may be found based on the location coordinate.

step 202: selecting a qualified DNA sequence of the sequence tagged site region meeting requirement for designing the capture probe.

The capturing efficiency of a chip may be affected by repeatability and GC content of DNA sequence, which may resulting in error capture. Thus, it is very important to select a qualified DNA sequence meeting requirement for designing the capture probe.

step 203: designing and synthesizing the capture probe based on the qualified DNA sequence.

To capture DNA fragment, the capture probe generally has a length of 60 to 150 bp, GC content between a range of 40% to 70%. When designing a probe for a DNA sequence of a same region, a plurality of probes are required for covering the entire DNA sequence, besides an overlap presents between two of the capture probes, in which the overlap general has a length of 20 bp.

In a specific embodiment, a location coordinate of DNA sequence in the sequence tagged site region of a chromosome is found in genomic database; and then the location coordinated is submitted to a company providing DNA capture service; and then designation and synthesis of the capture probe are completed by such company.

Preferably, as shown in Fig.3, the mixture library of the plurality of DNA samples is constructed by following steps:

step 301: constructing a plurality of DNA libraries for a plurality of single DNA samples, wherein each of the plurality of DNA libraries has a different adaptor respectively;

each of the plurality of DNA libraries has a different adaptor respectively, for distinguishing libraries derived from different DNA samples in the step of sequencing. Each of the plurality of DNA libraries has a different Index base sequence having a length of 6bp or 8bp at an end thereof.

step 302: mixing the plurality of DNA libraries with a predetermined ratio.

The plurality of DNA libraries may be mixed equally or with a predetermined ratio.

step 303: determining the resulting mixture as the mixture library, if the resulting mixture meets a requirement of quality control.

The mixture libraries of the plurality of DNA samples is subjecting to quantification, and to quality control to remove exogenous impurity.

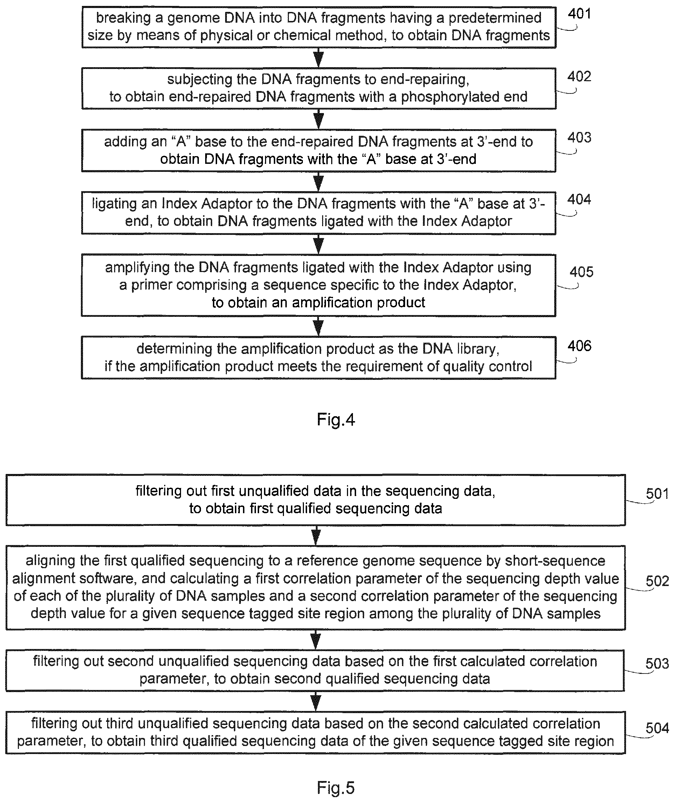

Preferably, as shown in Fig.4, the step of constructing a plurality of DNA libraries for a plurality of single DNA samples, for each of the plurality of single DNA samples, the DNA library is constructed by following steps:

step 401: breaking a genome DNA into DNA fragments having a predetermined size by means of physical or chemical method, to obtain DNA fragments.

In general, the obtained DNA fragments have a length of 200 to 300bp, and the captured probed has a length of about 80bp, as the length of the DNA fragments being as 200 to 300bp may resulting in a relative higher capture efficiency; in addition, the sequencing depth value is generally 200 to 300bp by PE sequencing after probe capturing.

step 402: subjecting the DNA fragments to end-repairing, to obtain end-repaired DNA fragments with a phosphorylated end;

step 403: adding an "A" base to the end-repaired DNA fragments at 3'-end to obtain DNA fragments with the "A" base at 3'-end;

step 404: ligating an Index Adaptor to the DNA fragments with the "A" base at 3'-end, to obtain DNA fragments ligated with the Index Adaptor;

step 405: amplifying the DNA fragments ligated with the Index Adaptor using a primer comprising a sequence specific to the Index Adaptor, to obtain an amplification product; and

step 406: determining the amplification product as the DNA library, if the amplification product meets the requirement of quality control.

Preferably, after the step of sequencing the captured DNA sequencing and prior to the step of analyzing the sequencing data, the method further comprises: subjecting the sequencing data to a quality control. As shown in Fig.5, the step of subjecting the sequencing data to the quality control further comprises:

step 501: filtering out first unqualified data in the sequencing data, to obtain first qualified sequencing data; preferably high-throughput sequencing technique may be Illumina Hiseq 2000 sequencing technique, or may be other high-throughput sequencing techniques existing in prior art.

step 502: aligning the first qualified sequencing to a reference genome sequence by short-sequence alignment software, and calculating a first correlation parameter of the sequencing depth value of each of the plurality of DNA samples and a second correlation parameter of the sequencing depth value for a given sequence tagged site region among the plurality of DNA samples;

step 503: filtering out second unqualified sequencing data based on the first calculated correlation parameter, to obtain second qualified sequencing data; and

step 504: filtering out third unqualified sequencing data based on the second calculated correlation parameter, to obtain third qualified sequencing data of the given sequence tagged site region.

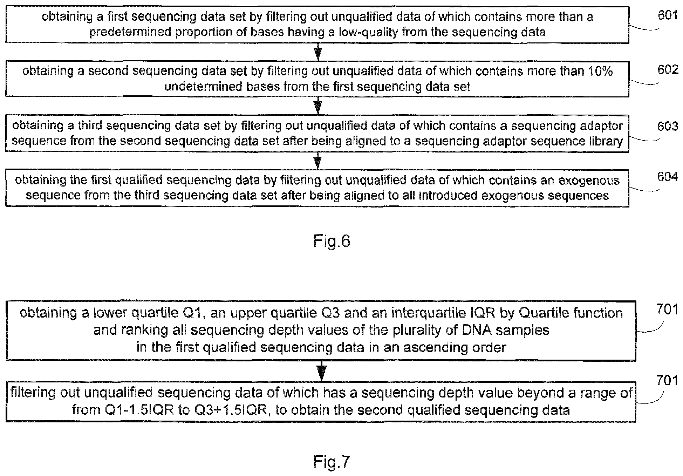

Preferably, as shown in Fig.6, in the step 501, the step of filtering out the first unqualified data in the sequencing data, to obtain the first qualified sequencing data further comprises:

step 601: obtaining a first sequencing data set by filtering out unqualified data of which contains more than a predetermined proportion of bases having a low-quality from the sequencing data. Different sequencing equipment uses different methods of calculating quality value, in which a standard for sequence with a low quality value may consult sequencing equipment companies or refer to a general standard in the art. In the current example, a formula for calculating a quality value used by HiSeq2000 sequencer of Illumina company is $Q = A - 64$, in which $Q$ is the sequencing quality value of a certain base, $A$ is an ASCII code of characters corresponding to the sequencing quality value of the certain base in an FQ file output by HiSeq2000 sequencer. In the current example, bases having a quality value lower than 5 is defined as a base with low quality value. If a sequence contains more than 50% bases having the low quality value, such sequence is determined as the first unqualified sequencing data, of which should be filtered out to obtain the first qualified sequencing data.

step 602: obtaining a second sequencing data set by filtering out unqualified data of which contains more than 10% undetermined bases from the first sequencing data set;

step 603: obtaining a third sequencing data set by filtering out unqualified data of which contains a sequencing adaptor sequence from the second sequencing data set after being aligned to a sequencing adaptor sequence library; and

step 604: obtaining the first qualified sequencing data by filtering out unqualified data of which contains an exogenous sequence from the third sequencing data set after being aligned to all introduced exogenous sequences.

In practice application, high-throughput sequencing technique may be Illumina Hiseq 2000 sequencing technique, or may be other high-throughput sequencing techniques existing in the art. Different sequencers or conditions may use different standard for filtering out unqualified sequencing data, for example, a certain standard may be used when sequencing by Illumina Hiseq 2000: a sequence containing more than 50% bases having a quality value lower than a certain threshold is regarded as an unqualified sequence, in which the certain threshold for low quality value depends on specific sequencing technique; a sequence containing more than 10% undetermined bases (as base N in a sequencing result by Illumina Hiseq 2000) is regarded as an unqualified sequence; except for sample adaptor sequence, those qualified sequencing data are aligned to other exogenous sequences introduced by experiment, such as various adaptor sequences. If the exogenous sequence presents, then an entire sequence containing the exogenous sequence is regarded as being unqualified.

Preferably, as shown in Fig.7, the step of filtering out the second unqualified sequencing data further comprises:

step 701: obtaining a lower quartile Q1, an upper quartile Q3 and an interquartile IQR by Quartile function and ranking all sequencing depth values of the plurality of DNA samples in the first qualified sequencing data in an ascending order.

In descriptive statistics, the quartiles of a ranked set of data values in an ascending order are the three points that divide the data set into four equal groups, each group comprising a quarter of the data. A first quartile (designated Q1), also known as "a smaller quartile", namely a lower quartile, equals to the 25%-th value in the ranked set of all data values. A second quartile (designated Q2), also known as a median, equals to the 50%-th value in the ranked set of all data values. A third quartile (designated Q2), also known as "a larger quartile" an upper quartile, equals to the 75%-th value in the ranked set of all data values. An interquartile range (designated IQR) is the difference between the upper quartile and the lower quartile. Each of the Q1, Q2 and Q3 is regarded as a demarcation point regardless values of such variances thereof, by which all of data values are divided into four equal parts. A trend of variances of the data values may be analyzed by comparing Q1 and Q3. The quartile has also been broadly applied in designing box-plot in statistics. The box-plot refers to a diagram composed of a box and two segments plotted with a set of data which are five statistical magnitudes (features), not only directly reflecting distribution of the set of data, but also being capable to analyze a plurality of data. Such five statistical magnitudes are maximum value, minimum value, median and two of quartiles.

step 702: filtering out unqualified sequencing data of which has a sequencing depth value beyond a range of from Q1-1.5IQR to Q3+1.5IQR, to obtain the second qualified sequencing data.

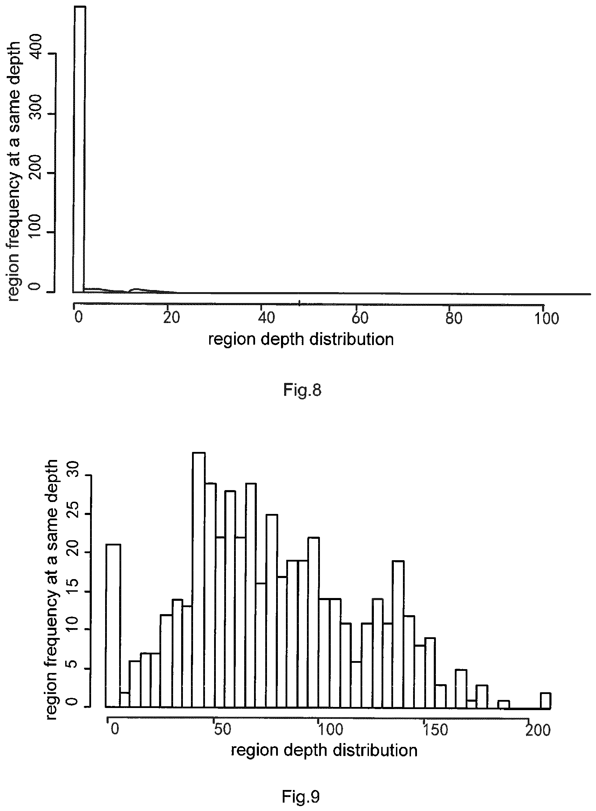

In an embodiment of the present disclosure, as shown in Fig.8 and Fig.9, X-coordinate indicates a region depth distribution; Y-coordinate indicates a region frequency for the same depth value. Fig.8 is a histogram showing a filtered out sample having an outlier of sequencing depth value. Fig.9 is a histogram showing a sample having normal sequencing depth value.

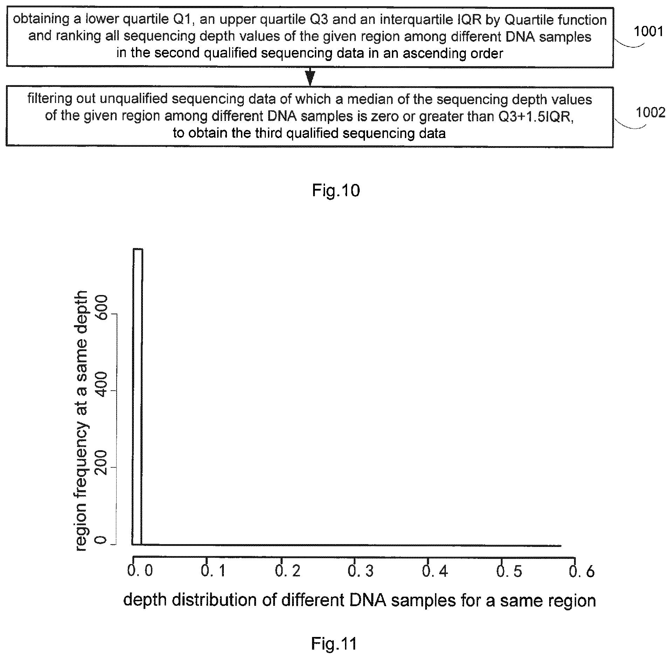

Preferably, as shown in Fig.10, the step of filtering out the third unqualified sequencing data further comprises:

step 1001: obtaining a lower quartile Q1, an upper quartile Q3 and an interquartile IQR by Quartile function and ranking all sequencing depth values of the given region among different DNA samples in the second qualified sequencing data in an ascending order; and

step 1002: filtering out unqualified sequencing data of which a median of the sequencing depth values of the given region among different DNA samples is zero or greater than Q3+1.5IQR, to obtain the third qualified sequencing data.

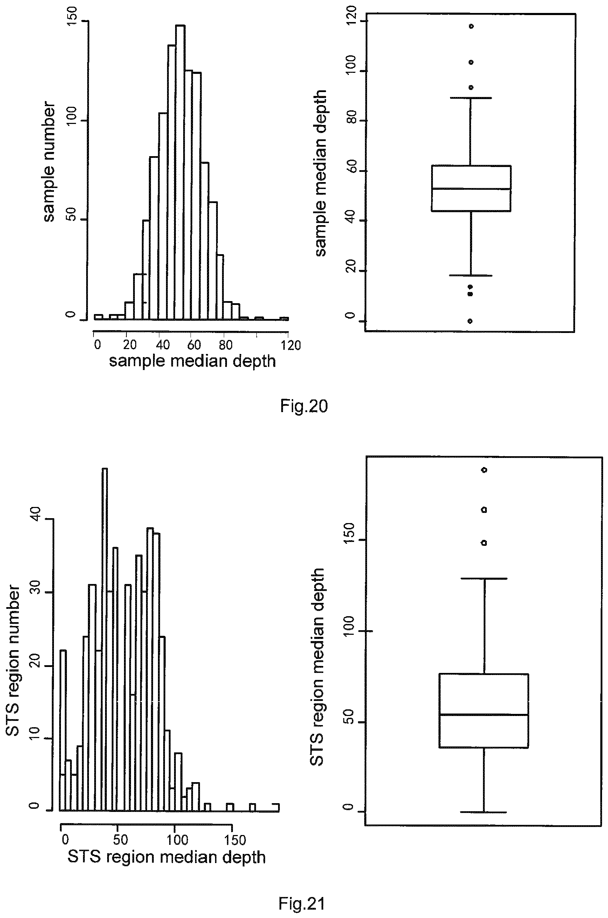

In another embodiment of the present disclosure, as shown in Fig.21, X-coordinate indicates depth distribution of the plurality of samples for a same region, Y-coordinate indicates region frequency with a same sequencing depth value. Fig.21 is a histogram showing a filtered out region having an outlier of sequencing depth value.

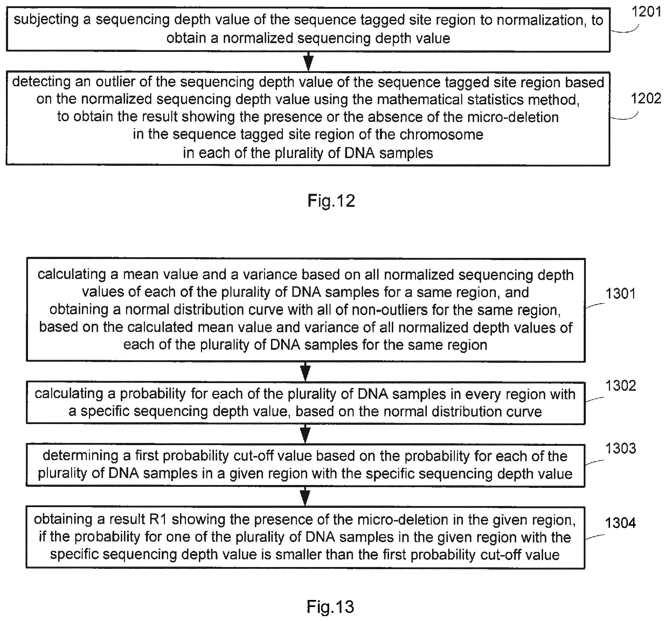

Preferably, as shown in Fig.12, the step of analyzing the sequencing data using the mathematical statistics test further comprises:

step 1201: subjecting a sequencing depth value of the sequence tagged site region to normalization, to obtain a normalized sequencing depth value. Preferably, the step of subjecting the sequencing depth value of the sequence tagged site region to normalization further comprises: dividing all of the sequencing depth values of each of the plurality of DNA samples for a same region by an average depth value of each of the plurality of DNA samples, to obtain the normalized depth values of the plurality of DNA samples for the same region.

step 1202: detecting an outlier of the sequencing depth value of the sequence tagged site region based on the normalized sequencing depth value using the mathematical statistics method, to obtain the result showing the presence or the absence of the micro-deletion in the sequence tagged site region of the chromosome in each of the plurality of DNA samples.

In a preferred embodiment, as shown in Fig.13, the step 1202 still further comprises:

step 1301: calculating a mean value and a variance based on all normalized sequencing depth values of each of the plurality of DNA samples for a same region;

step 1302: obtaining a normal distribution curve with all of non-outliers for the same region, based on the calculated mean value and variance of all normalized depth values of each of the plurality of DNA samples for the same region;

step 1303: calculating a probability for each of the plurality of DNA samples in every region with a specific sequencing depth value, based on the normal distribution curve;

step 1304: determining a first probability cut-off value based on the probability for each of the plurality of DNA samples in a given region with the specific sequencing depth value, and obtaining a result R1 showing the presence of the micro-deletion in the given region, if the probability for one of the plurality of DNA samples in the given region with the specific sequencing depth value is smaller than the first probability cut-off value.

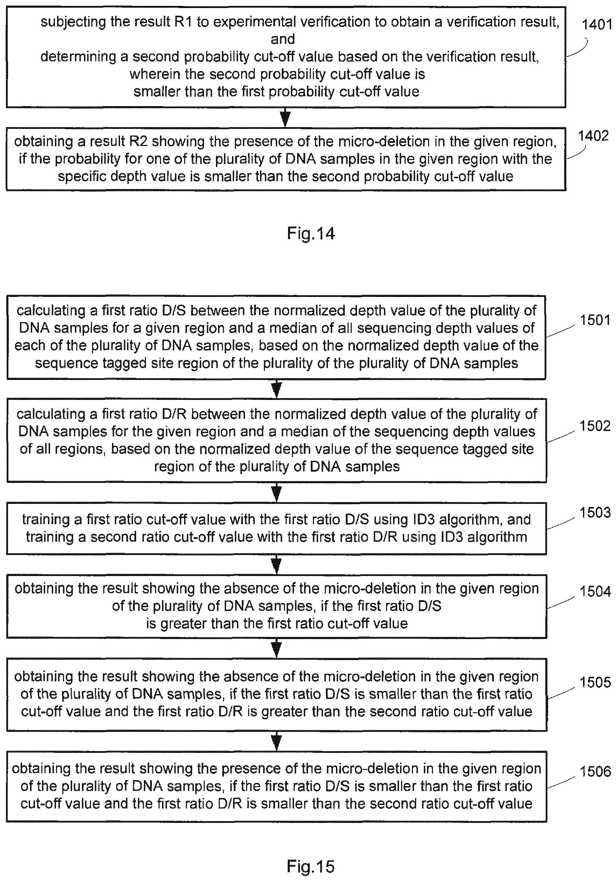

In another preferred embodiment, as shown in Fig.14, after the step 1304 the method further comprises:

step 1401: subjecting the result R1 to experimental verification, to obtain a verification result, and determining a second probability cut-off value based on the verification result, wherein the second probability cut-off value is smaller than the first probability cut-off value; and

step 1402: obtaining a result R2 showing the presence of the micro-deletion in the given region, if the probability for one of the plurality of DNA samples in the given region with the specific depth value is smaller than the second probability cut-off value.

In practical application, in the case that most samples have no micro-deletion, as for a certain region, a sequencing depth value of a sample having a micro-deletion should be an outlier of a normal distribution obtained with the samples having no micro-deletion. An appropriated mathematical statistics method is used to detect an outlier of the sequencing depth values for each region, by which a region having an outlier of the sequencing depth values is determined as a region having micro-deletion.

A method of determining whether a sequencing depth value for a given region is an outlier comprises: obtaining a normal distribution curve with all of non-outliers for the given region, based on calculated mean value and variance of all normalized depth values of each of the plurality of DNA samples for the given region, after filtering out outliers of the sequencing depth values; calculating a probability (p.value) for each of the plurality of DNA samples in every region with a specific sequencing depth value, based on the normal distribution curve; determining an appropriate first probability cut-off value (p.value-cutoff) for these probabilities; and obtaining a result R1 showing the presence of the micro-deletion in the given region, if the probability for one of the plurality of DNA samples in the given region with the specific sequencing depth value is smaller than the first probability cut-off value.

A method of determining the probability cut-off value may comprise: calculating the probability (p.value) for each of the plurality of DNA samples in every region with a sequencing depth value in the normal distribution according to the above-mentioned method; determining the presence or the absence of micro-deletion within more sample regions under a relaxed cut-off value; and subjecting these sample regions to experimental verification, to determine the presence or the absence of micro-deletion. A method of experimental verification may comprise: designing an appropriated probe; subjecting DNA of such sample regions to PCR reaction; determining whether PCR amplification product is normal, to determine the presence or the absence of micro-deletion. After obtaining information showing whether micro-deletion presents in such sample regions, an appropriate probability cut-off value may be selected, to have data with best false negative value and false positive value.

In a further preferred embodiment, as shown in Fig.15, the step of detecting the outlier of the sequencing depth value of the sequence tagged site region further comprises:

step 1501: calculating a first ratio D/S between the normalized depth value of the plurality of DNA samples for a given region and a median of all sequencing depth values of each of the plurality of DNA samples, based on the normalized depth value of the sequence tagged site region of the plurality of the plurality of DNA samples;

step 1502: calculating a first ratio D/R between the normalized depth value of the plurality of DNA samples for the given region and a median of the sequencing depth values of all regions, based on the normalized depth value of the sequence tagged site region of the plurality of DNA samples;

step 1503: training a first ratio cut-off value with the first ratio D/S using ID3 algorithm, and training a second ratio cut-off value with the first ratio D/R using ID3 algorithm;

step 1504: obtaining the result showing the absence of the micro-deletion in the given region of the plurality of DNA samples, if the first ratio D/S is greater than the first ratio cut-off value;

step 1505: obtaining the result showing the absence of the micro-deletion in the given region of the plurality of DNA samples, if the first ratio D/S is smaller than the first ratio cut-off value and the first ratio D/R is greater than the second ratio cut-off value; and

step 1506: obtaining the result showing the presence of the micro-deletion in the given region of the plurality of DNA samples, if the first ratio D/S is smaller than the first ratio cut-off value and the first ratio D/R is smaller than the second ratio cut-off value.

In practical applications, when a plurality of samples and regions need to be detected, and a proportion of sample regions having micro-deletion is relative smaller, the ratio between sequencing depth value of the region having micro-deletion and the median of all sequencing depth values of the plurality of DNA samples is usually smaller, and the ratio between sequencing depth value of the region having micro-deletion and the median of sequencing depth values of all regions is also usually smaller. Thus, a method based on decision tree using an appropriate parameter may be used in verifying an outlier of sequencing depth value.

Specific steps of the method based on decision tree may comprise: calculating a first ratio between a sequencing depth value of a sample region to be tested and a median of all sequencing depth values of a plurality of DNA samples; determining a result showing an absence of micro-deletion if the first ratio is greater than a first certain cut-off value; calculating a second ratio between the sequencing depth value of the sample region to be tested and a median of the median of the sequencing depth values of all regions, if the first ratio is smaller than the first certain cut-off value; determining the result showing an absence of micro-deletion if the second ratio is greater than a second certain cut-off value, and determining a result showing a presence of micro-deletion if the second ratio is smaller than the second certain cut-off value.

Determination of a cut-off value in decision tree test is calculated by Iterative Dichotomiser 3 (ID3) algorithm of decision tree (Mitchell, Tom M. Machine Learning. McGraw-Hill, 1997). Kernel of the ID3 algorithm comprises: during selecting an attribute among internodes of the decision tree, information gain is taken as a standard for attribute selection, such that when subjecting each of non-leaf node to tests, the maximum class information regarding the tested record may be obtained.

Basic ideas for the ID3 algorithm of decision tree comprise:

step 1, selecting one attribute as a root node of the decision tree, and creating branches with all values of the attribute;

step 2: classifying trained data using the created tree, in which

if all examples of one leaf node belong to a same class, such leaf node is labeled with the same class; then if all leaf nodes are labeled with classes, the algorithm is terminated;

step 3: if there is still a leaf node without being labeled, an attribute never presenting in a path from the leaf node to the root node is used to label such leaf node; and continuously creating branches with all values of such attribute; repeating step 2 of the algorithm.

In step 1, selecting different attributes may create different decision tree, thus selecting an appropriate attribute may create a simple decision tree. In ID3 algorithm, an attribute is generally selected by a heuristic information-based method. The attribute selected by the heuristic method may comprise highest amount of information, resulting in a decision tree having minimum branches. In the present disclosure, there are two distributes to be selected, namely, a ratio T1 (D/S) between the sequencing depth value of sample region and a median of sequencing depth value of sample region, a ratio T2 (D/R) between sequencing depth value of the plurality of DNA sample for a same region and a median of sequencing depth value of the plurality of DNA samples for a same region.

Attributes with maximum information are determined by information gain. A method of calculating information gain may comprise:

D is assumed a division of training set by classes, and an entropy of D is represented by

$$info(D) = -\sum_{i=1}^{m} p_i log_2(p_i)$$

, in which $p_i$ represents a probability of the i-th class presenting in training set, which may be estimated by means of dividing the number of training set belonging to such class by the total number of all training set. A practical meaning of the entropy refers an average information amount required for class labeling of the training set in D.

Assuming that the training set in D are divided by an attribute A, then expecting information of dividing D by A

is $$info_A(D) = \sum_{j=1}^{v} \frac{|D_j|}{|D|} info(D_j)$$ , while the information gain is $guin(A) = info(D) - info_A(D)$.

ID3 algorithm calculates a gain rate of each attibute at each required division, and divids based on an attribute having the maximum gain rate.

An example of detecting unreal account in a community was shown below to illustrate how to create a decision tree using the ID3 algorithm. For simplicity, a training set was assumed containing 10 elements, which was shown in Table 1.

Table 1 detection of unreal account in a community

| Log density | Friends density | whether or not using a real head portrait | whether or not being a real account |
|---|---|---|---|
| s | s | no | no |
| s | l | yes | yes |
| l | m | yes | yes |
| m | m | yes | yes |
| l | m | yes | yes |
| m | l | no | yes |
| m | s | no | no |
| l | m | no | yes |
| m | s | no | yes |
| s | s | yes | no |

s, m and l respectively represents small, medium, and large. Assuming that L, F, H and R respectively represent log density, friend density, whether or not using a real head portrait and whether or not being a real account. Gain information of every attribute was calculated below.

$$info(D) = -0.7log_2 0.7 - 0.3log_2 0.3 = 0.7 * 0.51 + 0.3 * 1.74 = 0.879$$
$$gain(L) = 0.879 - 0.603 = 0.276$$

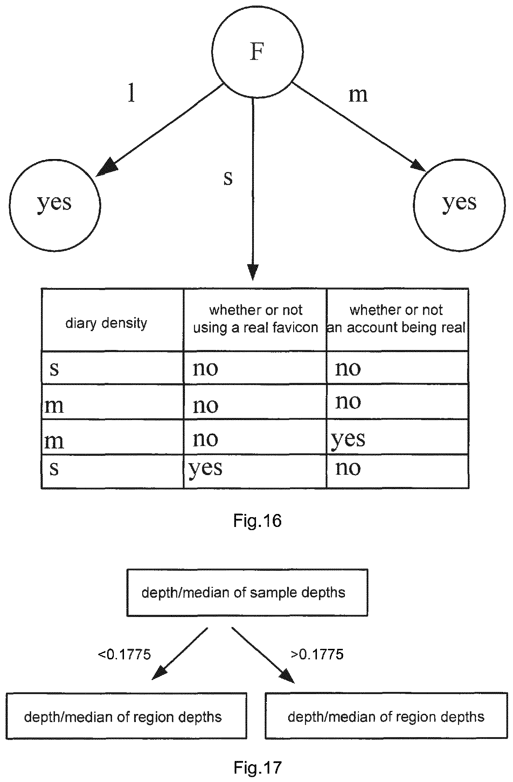

Thus, the gain information of log density was 0.276. The gain information of H and F respectively were 0.033 and 0.553 using the same method. As F had the maximum information gain, F was selected as the attribute for the first division, a result after the first division was shown as Fig.16.

While to solve data having consecutiveness attribute being similar as T1 and T2, the data was subjected to discretization firstly. A simple method thereof was dividing values of the attribute A into two parts: one part Ai contains values the attribute A being smaller than N, and the other part Ai contains values the attribute A being greater than N. For any one of attributes, all values in one data set is limited. Assuming that the values of the attribute was (v1, v2, ...,vn). Then in such data set, there were totally n-1 parts of division values, based on which a decision tree was constructed.

Specific steps of discretization may contain:

1) finding the minimum value (MIN) of the consecutive attribute, finding the maximum value MAX of the consecutive attribute;
2) determining N equally-divided points Ai in an interval (MIN, MAX), which were respectively Ai=MIN+((MAX-MIN)/N)*i, in which i=1, 2, 3......;
3) calculating a gain value taking (MIN, Ai) and (Ai, MAX) as the interval value respectively, and comparing the calculated gain values;
4) selecting Ak having the maximum gain value as a break point of the consecutive attribute, and determining two intervals of the attribute as (MIN, Ak) and (Ak, MAX).

In an example of the present disclosure, specific steps of determining the cut-off value based on the decision tree comprised:

Firstly, subjecting selected plurality of samples to PCR verification of partial region, to obtain the result showing presence of the micro-deletion, in which "+" represented that the result showing presence of the micro-deletion was positive; "-" represented that the result showing absence of the micro-deletion was negative

calculating entropy based on a formula of Info(D)=-(P+)log2 (P+)-(P-)log2(P-), in which p+ (P-) represented a probability of presence (absence) of the micro-deletion in whole training group, which could be estimated by dividing element amount belonging to such class by the total amount of elements in the whole training sets. The actual meaning of entropy referred to an average information amount required for class labeling of training sets in D.

Secondly, calculating a ratio T1 between normalized sequencing depth value of sample region and the median of all sequencing depth values of the plurality of DNA samples;

ranking obtained ratio T1 in an ascending order, to obtain V1, V2, V3, ..., Vn;

taking a mean value of a pair of consecutive ranked values as a cut-off value a1'=(V1+V2)/2, a2'=(V2+V3)/2, ..., (n-1)'=(Vn-1+Vn)/2;

obtaining the minimum cut-off value a1' and the maximum cut-off value a(n-1)'.

Thirdly, calculating a ratio T2 between normalized sequencing depth value of sample region and the median of sequencing depth values of all regions;

ranking obtained ratio T2 in an ascending order, to obtain U1, U2, U3, ..., Un;

taking a mean value of a pair of consecutive ranked values as a cut-off value b1'=(U1+U2)/2, b2'=(U2+U3)/2, ..., b(n-1)'=(Un-1+Un)/2;

obtaining the minimum cut-off value b1' and the maximum cut-off value b(n-1)'.

Fourthly, calculating entropies of different divided values, a1', a2', ..., obtained by cycling based on:

$$info_A(D) = \sum_{j=1}^{v} \frac{|D_j|}{|D|} info(D_j)$$,

while the information gain thereof was:

$$gain(A) = info(D) - info_A(D).$$

Fifth, calculating entropies of different divided values, b1', b2', ..., obtained by cycling based on:

$$info_A(D) = \sum_{j=1}^{v} \frac{|D_j|}{|D|} info(D_j)$$,

while the information gain thereof was:

$$gain(A) = info(D) - info_A(D).$$

Sixth, comparing the groups of the maximum gains of information gain, in which the attribute (a) having the larger value was taken as a root;

classifying information based on a cut-off value having the maximum cut-off value of gain value, which was shown as Fig.17.

Seventh, calculating the maximum gain by dividing sequencing depth values of two groups data by a median of sequencing depth values in the region respectively according to the above-mentioned steps;

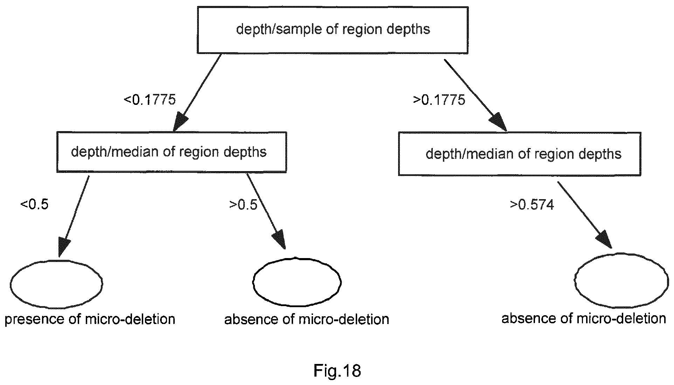

classifying information based on the cut-off value having the maximum cut-off value of gain value, which was shown as Fig.18, i.e. the final determined decision tree of the present disclosure.

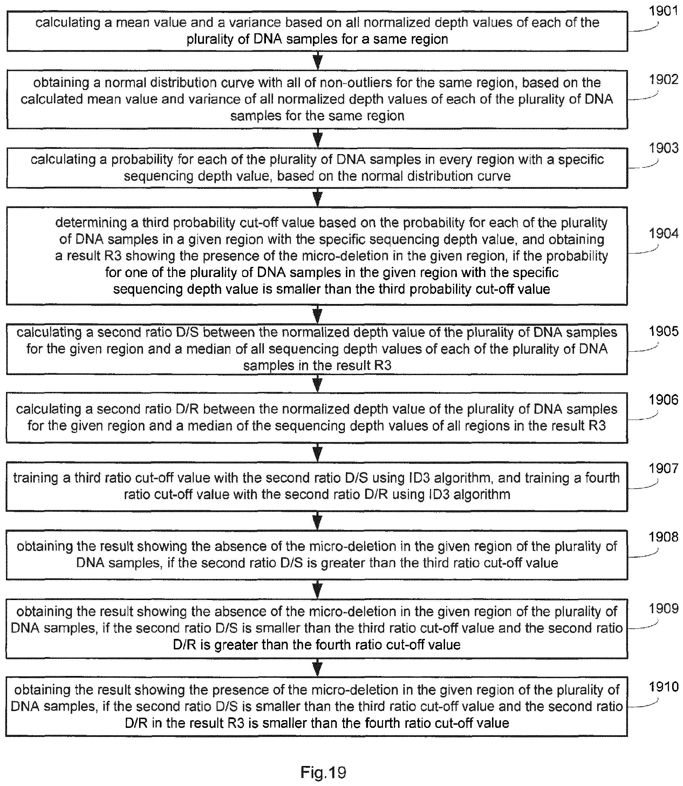

In a further preferred embodiment, as shown in Fig.19, the step of detecting the outlier of the sequencing depth value of the sequence tagged site region further comprises:

step 1901: calculating a mean value and a variance based on all normalized depth values of each of the plurality of DNA samples for a same region;

step 1902: obtaining a normal distribution curve with all of non-outliers for the same region, based on the calculated mean value and variance of all normalized depth values of each of the plurality of DNA samples for the same region;

step 1903: calculating a probability for each of the plurality of DNA samples in every region with a specific sequencing depth value, based on the normal distribution curve;

step 1904: determining a third probability cut-off value based on the probability for each of the plurality of DNA samples in a given region with the specific sequencing depth value, and obtaining a result R3 showing the presence of the micro-deletion in the given region, if the probability for one of the plurality of DNA samples in the given region with the specific sequencing depth value is smaller than the third probability cut-off value;

step 1905: calculating a second ratio D/S between the normalized depth value of the plurality of DNA samples for the given region and a median of all sequencing depth values of each of the plurality of DNA samples in the result R3;

step 1906: calculating a second ratio D/R between the normalized depth value of the plurality of DNA samples for the given region and a median of the sequencing depth values of all regions in the result R3;

step 1907: training a third ratio cut-off value with the second ratio D/S using ID3 algorithm, and training a fourth ratio cut-off value with the second ratio D/R using ID3 algorithm;

step 1908: obtaining the result showing the absence of the micro-deletion in the given region of the plurality of DNA samples, if the second ratio D/S is greater than the third ratio cut-off value;

step 1909: obtaining the result showing the absence of the micro-deletion in the given region of the plurality of DNA samples, if the second ratio D/S is smaller than the third ratio cut-off value and the second ratio D/R is greater than the fourth ratio cut-off value; and

step 1910: obtaining the result showing the presence of the micro-deletion in the given region of the plurality of DNA samples, if the second ratio D/S is smaller than the third ratio cut-off value and the second ratio D/R in the result R3 is smaller than the fourth ratio cut-off value.

In practical applications, to more accurately detect a region having micro-deletion, a strategy combining the above two method may be used, which comprises: predetermining a relaxed p.value - cut-off value, and filtering resulting data based on decision tree, to obtain the result showing presence of micro-deletion in the sequence tagged site region of the chromosome.

The method of detecting the micro-deletion in the sequence tagged site region of the chromosome and the apparatus thereof, by which obtains the capture probe covering an entire sequence tagged site region of a chromosome and captures DNA sequence of the sequence tagged site region from a plurality of DNA samples after being hybridized with a mixture library of the plurality of DNA samples, may efficiently and accurately detect reported or unreported micro-deletions in a sequence tagged site-related region of a chromosome with a large amount of DNA samples; besides the step of analyzing by the mathematical statistics method in the present disclosure may be based on a normal distribution, or may be based on an analysis method of decision tree, or may be based on a combination thereof, followed by an experimental verification, such process of information analysis is scientific, stable, with a high sensitivity, a low false positive rate, which may be used in effective analysis for the micro-deletions.

Reference will be made in detail to examples of the present disclosure. It would be appreciated by those skilled in the art that the following examples are explanatory, and cannot be construed to limit the scope of the present disclosure. If the specific technology or conditions are not specified in the examples, a step will be performed in accordance with the techniques or conditions described in the literature in the art (for example, referring to J. Sambrook, et al. (translated by Huang PT), Molecular Cloning: A Laboratory Manual, 3rd Ed., Science Press) or in accordance with the product instructions. If the manufacturers of reagents or instruments are not specified, the reagents or instruments may be commercially available, for example, from Illumina.

The current example took an example of detecting a micro-deletion in a sequence tagged site region of chromosome Y, but not limited with chromosome Y. A total 11 sample including 10 infertility samples and 1 health human sample were used in the current example, which were subjected to hybridization with a Nimblegen (Roche) chip after being library-constructed together. The number of samples in the current example was used to illustrated the present disclosure, but not limited to the number of samples for one experiment.

Reagents used in the current example were shown as Table 2.

Table 2. Reagents used in the current example of the present disclosure

| Reagent | Model | Manufacturer |
| --- | --- | --- |
| QIAquick PCR Purification Kit | 28106 | QIAGEN |
| 10×Polynucleotide Kinase Buffer | B904 | Enzymatics |

(continued)

| Reagent | Model | Manufacturer |
|---|---|---|
| dNTP Solution Set | N201L | Enzymatics |
| T4 DNA Polymerase | P708L | Enzymatics |
| Klenow Enzyme | P706L | Enzymatics |
| T4 Polynucleotide Kinase | Y904L | Enzymatics |
| 10× blue buffer | B011 | Enzymatics |
| 25 mM dATP | 28406501V | GE |
| Klenow (3'-5' exo-) | P701-LC-L | Enzymatics |
| 2× Rapid ligation buffer | B101 | Enzymatics |
| T4 DNA Ligase (Rapid) | L603-HC-L | Enzymatics |
| PE/PE Index Adapter oligo mix (40 μM) | | Invitrogen |
| MiniElute PCR Purification Kit | 28006 | QIAGEN |
| Platinum® Pfx DNA Polymerase | C11708-013 | Invitrogen |
| PCR Primer PE 1.0/PE Index Primer 1.0 (10 pM) | | Invitrogen |
| PCR Primer PE 2.0/PE Index Primer 2.0 (index, 10 pM) | | Invitrogen |
| Multiplexing Sample Preparation Oligonucleotide Kit | PE-400-1001 | PE-400-1001 |
| 2×SC Hybridiation Buffer | 5340721001 | Nimblegen |
| SC Hybridiation Component A | 5340721001 | Nimblegen |
| Phusion Mix | F-531L | NEB |
| Multiplexing Sequencing Primers and Phix Control Kit | PE-400-1002 | Illumina |
| Cot-1 DNA | 15279-011 | Invitrogen |

I. fragmentation of genome DNA

The step of constructing the plurality of DNA libraries for the plurality of single DNA samples included: breaking the genome DNA into DNA fragments having a predetermined size by means of a physical or chemical method, to obtain broken DNA fragments.

Yan Huang genome DNA (http://yh.genomics.org.cn/) having a weight of 3 μg without protein, RNA contamination or degradation was taken as an initial material, which was broken using an ultrasound fragmentation equipment Covaris-S2 (Covaris, US). Parameters used for fragmentation were shown as Table 3.

Table 3 Parameter setting of the ultrasound fragmentation equipment Covaris-S2

| treatment 1 | Loading ratio (%) | 10 |
|---|---|---|
| | Intensity | 10 |
| | cycle/impulse | 1000 |
| | time (s) | 60 |
| treatment 2 | time (s) | 0 |
| treatment 3 | time (s) | 0 |
| treatment 4 | time (s) | 0 |
| cycles | | 4 |

The fragmented sample was subjected to quality test by electrophoresis, then the qualified sample was purified by QIAquick PCR Purification Kit, and then the purified sample was dissolved in 32 μL of an elution buffer. The major bands of qualified DNA are centralized between 200 bp to 300 bp.

II. End-repairing of DNA fragments

The step of constructing the plurality of DNA libraries for the plurality of single DNA samples included: subjecting the DNA fragments to end-repairing, to obtain end-repaired DNA fragments with a phosphorylated end.

DNA obtained from the previous step was formulated into a reaction system for end-repairing according to the following composition in a 1.5 mL centrifuge tube shown in Table 4.

Table 4 Reaction system for end-repairing

| DNA sample | 75 μL |
|---|---|
| 10× Polynucleotide Kinase Buffer | 10 μL |
| dNTP Solution Set (10 mM each) | 4 μL |
| T4 DNA Polymerase | 5 μL |
| Klenow Fragment | 1 μL |
| T4 Polynucleotide Kinase | 5 μL |
| Total volume | 100 μL |

After gentle mixing, the tube containing 100 μL of the above reaction mixture was placed on a Thermomixter (Eppendorf) set at 20°C, and allowed the reaction for 30 min. After the reaction was completed, the obtained sample was purified using QIAquick PCR Purification Kit (Qiagen). Then, the purified sample was dissolved in 32 μL of ddH$_2$O for sufficient dissolve.

III. Adding a base "A" at the end-repaired DNA fragments

The step of constructing the plurality of DNA libraries for the plurality of single DNA samples included: adding an "A" base to the end-repaired DNA fragments at 3'-end to obtain DNA fragments with the "A" base at 3'-end.

DNA obtained from the previous step was formulated into a reaction system for adding a base A according to the following composition in a 1.5 mL centrifuge tube shown in Table 5.

Table 5 Reaction system for adding a base A to the end-repaired DNA fragments

| DNA | 32 μL |
|---|---|
| 10× blue buffer | 5 μL |
| dATP (1mM) | 10 μL |
| Klenow (3'-5' exo-) | 3 μL |
| Total volume | 50 μL |

After gentle mixing, the tube containing 50 μL of the above reaction mixture was placed on a Thermomixter (Eppendorf) set at 37°C, and allowed the reaction for 30 min. After the reaction was completed, the obtained sample was purified using QIAquick PCR Purification Kit (Qiagen). Then, the purified sample was dissolved in 15 μL of ddH$_2$O for sufficient dissolve.

IV. Ligating an index adaptor

The step of constructing the plurality of DNA libraries for the plurality of single DNA samples included: ligating an Index Adaptor to the DNA fragments with the "A" base at 3'-end, to obtain DNA fragments ligated with the Index Adaptor.

DNA obtained from the previous step was formulated into a reaction system for ligating an index adaptor according to the following composition in a 1.5 mL centrifuge tube shown in Table 6.

Table 6. Reaction system for ligating an index adaptor

| DNA added with a base "A" | 15 $\mu$L |
|---|---|
| 2$\times$ Rapid ligation buffer | 25 $\mu$L |
| PE/PE index Adapter oligo mix (40 $\mu$M) | 5 $\mu$L |
| T4 DNA Ligase (Rapid) | 5 $\mu$L |
| Total volume | 50 $\mu$L |

After gentle mixing, the tube containing 50 $\mu$L of the above reaction mixture was placed on a Thermomixter (Eppendorf) set at 20°C, and allowed the reaction for 15 min. After the reaction was completed, the obtained sample was purified using MiniElute PCR Purification Kit (Qiagen). Then, the purified sample was dissolved in 25 $\mu$L of the elution buffer.

V. PCR prior to hybridization

The step of constructing the plurality of DNA libraries for the plurality of single DNA samples included: amplifying the DNA fragments ligated with the Index Adaptor using a primer comprising a sequence specific to the Index Adaptor, to obtain an amplification product.

Taking the DNA sample in the step IV as an template, the PCR amplification was performed by taking the sequence containing the adaptor as the primer, in which the amplification system was shown as Table 7.

Table 7. PCR amplification system

| DNA containing adaptor sequence | 25 $\mu$L |
|---|---|
| 10x pfx amplification buffer | 10 $\mu$L |
| MgSO$_4$ (50mM) | 4 $\mu$L |
| dNTP mix (10mM) | 4 $\mu$L |
| Platinum® Pfx DNA Polymerase | 2 $\mu$L |
| PCR Primer PE 1.0/PE Index Primer 1.0 (10pM) | 10 $\mu$L |
| PCR Primer PE 2.0/PE Index Primer 2.0 (index, 10pM) | 10 $\mu$L |
| ddH$_2$O | 35 $\mu$L |
| Total volume | 100 $\mu$L |

Condition of PCR reaction:

```
94 °C    2 min  ⌐
94 °C    15 s    |
62 °C    30 s    ⟩   4 cycles
72 °C    30 s    |
72 °C    5 min  ⌐
```

The obtained amplification product was purified using QIAquick PCR Purification Kit with 30 $\mu$L of elution buffer.

VII. Constructing a mixture library

The mixture library of the plurality of DNA samples was constructed by following steps: mixing the plurality of DNA libraries with a predetermined ratio, such as with equal amount. In practical applications, the appropriate ratio could be determined according to requirement for constructing the mixture library.

VII. Hybridizing a target region with the capture probe

The method of detecting the micro-deletion in the sequence tagged site region of a chromosome included: hybridizing the capture probe with a mixture library of a plurality of DNA samples, to capture the DNA sequence of the sequence tagged site region from the plurality of DNA samples.

1) 450 $\mu$g of COT-1 DNA, 3 $\mu$g of the mixture library from the step VI, 1 nmol of Index-adpater1-block and Index-adpater2-block (Multiplexing Sample Preparation Oligonucleotide Kit, Illumina) were added into a 1.5 mL centrifuge tube. The tube containing the above mixture in 1) was placed on a SpeedVac (Thermo) setting at 60°C.

2) The centrifuge tube containing dried mixture was added with 11.2 $\mu$L of purified water for sufficient dissolve DNA, followed by adding with 18.5 $\mu$L of Hybridiation Buffer and 7.3 $\mu$L of SC Hybridiation. After mixed the obtained mixture was placed on a hybridizer (Nimblegen) setting at 95°C, and allowed DNA denature for 10 min.

3) After been shaken, the sample obtained from 2) was centrifuged at the maximum speed for 30 second, and then placed on a hybridizer (Nimblegen) setting at 42°C for preparing hybridization.

4) Hybridization method could refer to the chip hybridization method of NimbleGen Company (NimbleGen Arrays User's Guide, Version 3.1, 7 Jul 2009, Roche NimbleGen, Inc.). The amount of sample to be tested was 35 $\mu$L, the hybridization was performed at 42 °C for 64 to 72 hours. The hybridized product was eluted with 900 $\mu$L of 160mM NaOH, and the eluted product was purified using MinElute PCR Purification Kit, finally the purified product was eluted with 80 $\mu$L of Elution Buffer.

VIII. PCR after capturing

The captured library was subjected to PCR amplification with a reaction system of 150 $\mu$L of Phusion Mix, 4.2 $\mu$L of each of upstream primer and downstream primer (Multiplexing Sequencing Primers and Phix Control Kit), 80 $\mu$L of the purified product in the elution buffer and 85 $\mu$L of ddH$_2$O. After divided into 6 tubes, the tubes containing the mixture were subjected to PCR amplification for 16 cycles. After PCR reaction, the 6 tubes containing amplified products were mixed together, and the mixed amplification product was subjecting to beads purification using QIAquick PCR Purification Kit. The purified product having a length of 300 to 450 bp was eluted with 50 $\mu$L of elution buffer.

VIIII. Library detection

Length size and content of the inserted fragments contained in the obtained library were detected by Bioanalyzer analysis system (Agilent, Santa Clara, USA). And concentration of the obtained library was accurately quantified by Q-PCR.

X. Sequencing and data analysis

The method of detecting the micro-deletion in the sequence tagged site region of the a chromosome included:

sequencing the captured DNA sequence of the sequence tagged site region to obtain sequencing data; and analyzing the sequencing data using a mathematical statistics method, to obtain a result showing a presence or an absence of the micro-deletion in the sequence tagged site region of the chromosome in each of the plurality of DNA samples.

The qualified library obtained in step VIIII was subjected to sequencing on computer, which could refer to HiSeq 2000 User Guide. Catalog # SY-940-1001 Part # 15011190 Rev B, Illumina.

Process of information analysis in the current example of the present disclosure was shown below:

1. receiving sequencing data obtained by high-throughput sequencing technique, and subjecting the sequencing data to quality control:

the step of subjecting the sequencing data to the quality control further comprised:

filtering out unqualified data in the sequencing data, to obtain qualified sequencing data.

In the current example of the present disclosure, Illumina Hiseq 2000 high-throughput sequencing technique was used. After been received, the sequencing data was subjected to filtration, to filter out unqualified sequence. The unqualified sequence met at least one criterion:

containing more than 50% bases having a sequencing quality lower than 5;
containing more than 10% bases being as N;
containing a sequencing adaptor sequence after being aligned to a sequencing adaptor sequence library.

2. Calculation of sequencing depth value of sample region:
the step of subjecting the sequencing data to the quality control further comprised:

aligning the first qualified sequencing to a reference genome sequence by short-sequence alignment software, and calculating a first correlation parameter of the sequencing depth value of each of the plurality of DNA samples and a second correlation parameter of the sequencing depth value for a given sequence tagged site region among the plurality of DNA samples;
In the current example, the sequencing data obtained by the high-throughput sequencing technique was aligned to a human genome reference sequence using SOAPaligner. HG19 (http://genome.ucsc.edu/) was used as the human genome reference sequence. The sequencing depth value of sample region was subjected to statistical calculation.

3. Calculating sequencing depth value for each sample
the step of subjecting the sequencing data to the quality control further comprised:
filtering out second unqualified sequencing data based on the first calculated correlation parameter, to obtain second qualified sequencing data.
The step of filtering out the second unqualified sequencing data further comprised:

obtaining a lower quartile Q1, an upper quartile Q3 and an interquartile IQR by Quartile function and ranking all sequencing depth values of the plurality of DNA samples in the first qualified sequencing data in an ascending order; and
filtering out unqualified sequencing data of which has a sequencing depth value beyond a range of from Q1-1.5IQR to Q3+1.5IQR, to obtain the second qualified sequencing data.

As shown in Fig.20, a sample having a sequencing data being as the outlier was filtered out. In Fig.20, the left panel was a histogram a median of sequencing depth value of a sample, the right panel was a box chart showing a median of sequencing depth value of a sample. It could be seen from that the median of the sequencing depth values of the plurality of DNA samples mainly concentrated within 42X to 60X.
4. Calculating the sequencing depth value for every region
The step of subjecting the sequencing data to a quality control further comprised:

filtering out third unqualified sequencing data based on the second calculated correlation parameter, to obtain third qualified sequencing data of the given sequence tagged site region.
the step of filtering out the third unqualified sequencing data further comprised:

obtaining a lower quartile Q1, an upper quartile Q3 and an interquartile IQR by Quartile function and ranking all sequencing depth values of the given region among different DNA samples in the second qualified sequencing data in an ascending order; and
filtering out unqualified sequencing data of which a median of the sequencing depth values of the given region among different DNA samples is zero or greater than Q3+1.5IQR, to obtain the third qualified sequencing data.

As shown in Fig.21, the left panel was a histogram showing a median of sequencing depth value of a region; and the right panel was a box chart showing a median of sequencing depth value of the region. It could be seen from that the median of the sequencing depth values of sample region mainly concentrated within 35X to 75X.
5. after filtering out region or sample having an outlier of sequencing depth value, the data sequencing of every sample was subjected to normalization, in which all of the sequencing depth values of each of the plurality of DNA samples for a same region was divided by an average depth value of each of the plurality of DNA samples, to obtain the normalized depth values of the plurality of DNA samples for the same region. And then the normalized depth values were subjected to extreme value test by means of a normal distribution curve. i.e., the the step of detecting the outlier of the sequencing depth value of the sequence tagged site region further comprises:

calculating a mean value and a variance based on all normalized sequencing depth values of each of the plurality of DNA samples for a same region;
obtaining a normal distribution curve with all of non-outliers for the same region, based on the calculated mean value and variance of all normalized depth values of each of the plurality of DNA samples for the same region;

calculating a probability for each of the plurality of DNA samples in every region with a specific sequencing depth value, based on the normal distribution curve;

determining a first probability cut-off value based on the probability for each of the plurality of DNA samples in a given region with the specific sequencing depth value; and

obtaining a result R1 showing the presence of the micro-deletion in the given region, if the probability for one of the plurality of DNA samples in the given region with the specific sequencing depth value is smaller than the first probability cut-off value;

subjecting the result R1 to experimental verification, to obtain a verification result;

determining a second probability cut-off value based on the verification result, wherein the second probability cut-off value is smaller than the first probability cut-off value; and

obtaining a result R2 showing the presence of the micro-deletion in the given region, if the probability for one of the plurality of DNA samples in the given region with the specific depth value is smaller than the second probability cut-off value.

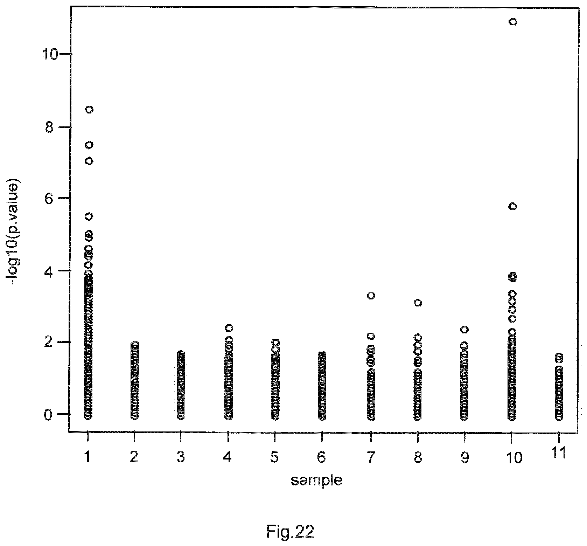

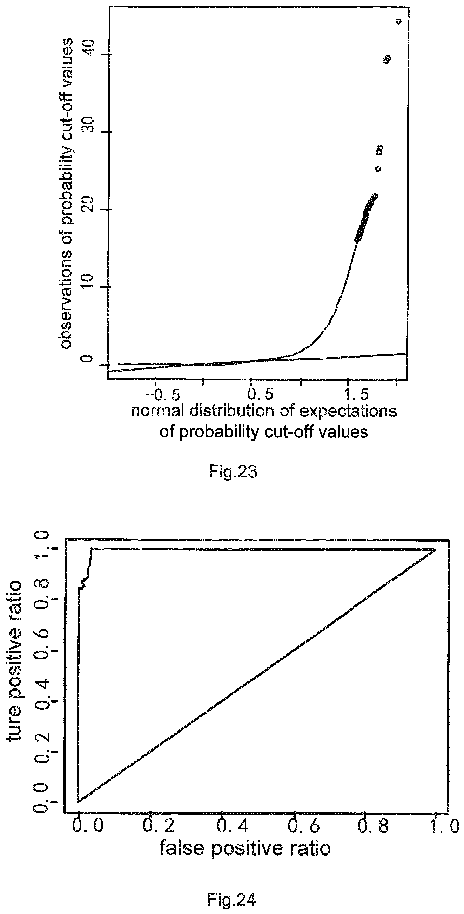

For a every region after being filtered out the outlier of sequencing depth value, selecting sequencing data of the plurality of DNA samples after being filtered out the outlier of sequencing depth value, the normal distribution curve with all of non-outliers for the same region was obtained based on the calculated mean value and variance of all normalized depth values of each of the plurality of DNA samples for the same region. The probability p.value for each of the plurality of DNA samples in every region with a specific sequencing depth value was obtained based on the normal distribution curve. As shown in Fig.22, the X-coordinate was sample, the Y-coordinate was -log10 (p-value); a diagram, shown as Fig.23, showed a result illustrating expectation values and observation values of the p-value. By such method, the probability of sequencing depth value of every DNA sample for every region was obtained. 20 sample regions having lower probabilities were subjected to experimental verification of PCR reaction. The probability cut-off value for detecting the micro-deletion was determined based on the verified result, and accuracy of detecting the micro-deletion was subjected to evaluation, by specific steps:

selecting partial region of some samples, which included the presence and the absence of the micro-deletion, as well as a region with a larger span of p.value;

designing primers for both ends of different regions, and subjecting the DNA samples to PCR amplification, and then subjecting the amplified product to electrophoresis analysis to obtain a result showing presence of the micro-deletion; and finally obtaining the cut-off value P.value having statistical significance. As shown in Table 8, to obtain true positive rate and false positive rate corresponding to different sequencing depth, a value area under curve (AUC) was obtained as up to 0.9968254 by verifying the method of detecting the high-throughput sequencing technique-based micro-deletion. As shown in Fig.24, the X-coordinate was the false positive rate, the Y-coordinate was the true positive rate. Fig.24 showed that different probabilities of cut-off values may be selected based on different true positive rates and false positive rates.

Table 8. Different true positive rates and false positive rates obtained by selecting different cut-off values after taking logarithm -log10 with the p.value

| cut-off ratio | true positive | false positive |
|---|---|---|
| 0 | 1 | 1 |
| 1 | 1 | 0.20952381 |
| 2 | 1 | 0.123809524 |
| 3 | 1 | 0.076190476 |
| 4 | 1 | 0.038095238 |
| 5 | 1 | 0.028571429 |
| 6 | 0.974358974 | 0.028571429 |
| 7 | 0.897435897 | 0.019047619 |
| 8 | 0.871794872 | 0.00952381 |
| 9 | 0.846153846 | 0.00952381 |

(continued)

| cut-off ratio | true positive | false positive |
|---|---|---|
| 10 | 0.846153846 | 0 |
| 11 | 0.820512821 | 0 |
| 12 | 0.512820513 | 0 |
| 13 | 0.512820513 | 0 |
| 14 | 0.512820513 | 0 |
| 15 | 0.512820513 | 0 |
| 16 | 0.512820513 | 0 |
| 17 | 0.512820513 | 0 |
| 18 | 0.512820513 | 0 |
| 19 | 0.512820513 | 0 |
| 20 | 0.487179487 | 0 |
| 21 | 0.256410256 | 0 |
| 22 | 0.256410256 | 0 |
| 23 | 0 | 0 |

6. In addition, the presence or the absence of micro-deletion in the sample region was determining by decision tree in view of the sequencing depth values of the sample region.

The step of detecting the outlier of the sequencing depth value of the sequence tagged site region further comprises:

calculating a first ratio D/S between the normalized depth value of the plurality of DNA samples for a given region and a median of all sequencing depth values of each of the plurality of DNA samples, based on the normalized depth value of the sequence tagged site region of the plurality of the plurality of DNA samples;

calculating a first ratio D/R between the normalized depth value of the plurality of DNA samples for the given region and a median of the sequencing depth values of all regions, based on the normalized depth value of the sequence tagged site region of the plurality of DNA samples;

training a first ratio cut-off value with the first ratio D/S using ID3 algorithm, and training a second ratio cut-off value with the first ratio D/R using ID3 algorithm;

obtaining the result showing the absence of the micro-deletion in the given region of the plurality of DNA samples, if the first ratio D/S is greater than the first ratio cut-off value;

obtaining the result showing the absence of the micro-deletion in the given region of the plurality of DNA samples, if the first ratio D/S is smaller than the first ratio cut-off value and the first ratio D/R is greater than the second ratio cut-off value; and

obtaining the result showing the presence of the micro-deletion in the given region of the plurality of DNA samples, if the first ratio D/S is smaller than the first ratio cut-off value and the first ratio D/R is smaller than the second ratio cut-off value.

In the current example, a relaxed cut-off value p.value $10^{-6}$ was predetermined, and obtained positive result was subjected to classification by decision tree, so as to further decrease the false positive rate, and then a site of micro-deletion in the sample region was obtained.

The step of detecting the outlier of the sequencing depth value of the sequence tagged site region further comprised:

calculating a mean value and a variance based on all normalized depth values of each of the plurality of DNA samples for a same region;

obtaining a normal distribution curve with all of non-outliers for the same region, based on the calculated mean value and variance of all normalized depth values of each of the plurality of DNA samples for the same region;

calculating a probability for each of the plurality of DNA samples in every region with a specific sequencing depth value, based on the normal distribution curve;

determining a third probability cut-off value based on the probability for each of the plurality of DNA samples in a given region with the specific sequencing depth value;

obtaining a result R3 showing the presence of the micro-deletion in the given region, if the probability for one of the plurality of DNA samples in the given region with the specific sequencing depth value is smaller than the third probability cut-off value;

calculating a second ratio D/S between the normalized depth value of the plurality of DNA samples for the given region and a median of all sequencing depth values of each of the plurality of DNA samples in the result R3;

calculating a second ratio D/R between the normalized depth value of the plurality of DNA samples for the given region and a median of the sequencing depth values of all regions in the result R3;

training a third ratio cut-off value with the second ratio D/S using ID3 algorithm, and training a fourth ratio cut-off value with the second ratio D/R using ID3 algorithm;

obtaining the result showing the absence of the micro-deletion in the given region of the plurality of DNA samples, if the second ratio D/S is greater than the third ratio cut-off value;

obtaining the result showing the absence of the micro-deletion in the given region of the plurality of DNA samples, if the second ratio D/S is smaller than the third ratio cut-off value and the second ratio D/R is greater than the fourth ratio cut-off value; and

obtaining the result showing the presence of the micro-deletion in the given region of the plurality of DNA samples, if the second ratio D/S is smaller than the third ratio cut-off value and the second ratio D/R in the result R3 is smaller than the fourth ratio cut-off value.

The present disclosure also provides a computer readable medium comprising an order, configured to perform the method according to the above-mentioned method by a processer, which is omitted for concise consideration.

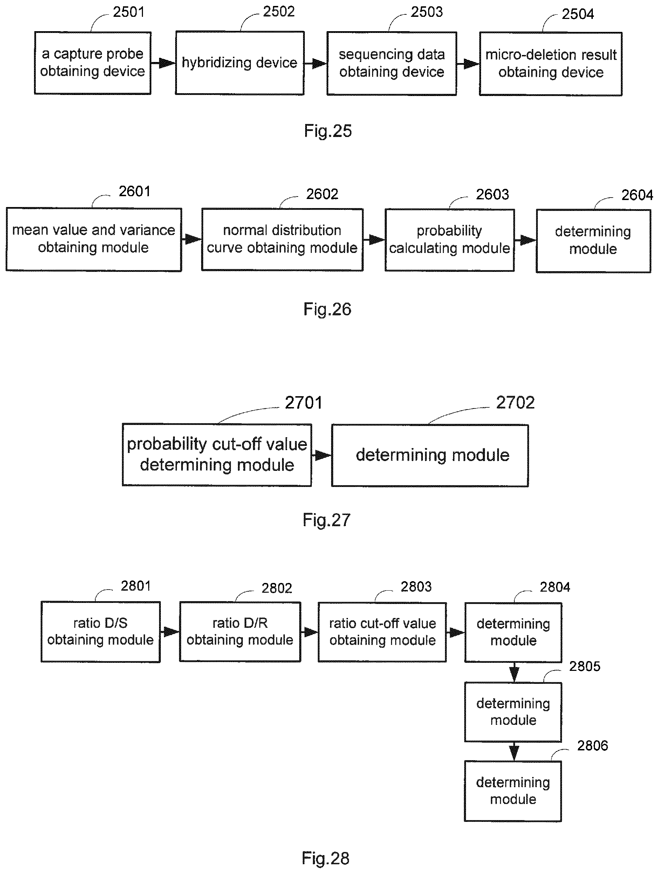

Fig.25 is a schematic diagram showing an apparatus for detecting a micro-deletion in a sequence tagged site region of a chromosome according to an embodiment of the present disclosure. As shown in Fig.25, the apparatus of the present disclosure may comprise:

a capture probe obtaining device 2501, a hybridizing device 2502, a sequencing data obtaining device 2503 and a micro-deletion result obtaining device 2504.

The capture probe obtaining device 2501 is for obtaining a capture probe corresponding to DNA sequence of the sequence tagged site region.

Sequence tagged-site (STS) is a short single-copy DNA sequence of known map location serving as a marker, which can be uniquely-mapped by PCR amplification, for producing a site for map-making, namely, detection with an order of a series of STS may make a map of a genomic region. A probe is a short single-strand DNA or RNA fragment (about 20 to 500 bp), for detecting a complementary nucleic acid sequence thereof.

The hybridizing device 2502 is for hybridizing the capture probe with a mixture library of a plurality of DNA samples, to capture the DNA sequence of the sequence tagged site region from the plurality of DNA samples.

The expression "mixture libraries" used herein refers to all DNA sequences for sequencing obtained by mixing a plurality of DNA libraries for a plurality of single DNA samples. Specific steps for constructing the mixture library are described above in details.

The sequencing data obtaining device 2503 is for sequencing the captured DNA sequence of the sequence tagged site region to obtain sequencing data.

The micro-deletion result obtaining device 2504 is for analyzing the sequencing data using a mathematical statistics method, to obtain a result showing a presence or an absence of the micro-deletion in the sequence tagged site region of the chromosome in each of the plurality of DNA samples.

Preferably, the capture probe obtaining device 2501 further comprises:

a region finding unit, for finding the DNA sequence of the sequence tagged site region within a genomic database (GDB);

a sequence selecting unit, for selecting a qualified DNA sequence of the sequence tagged site region meeting requirement for designing the capture probe; and

a capture probe obtaining unit, for designing and synthesizing the capture probe based on the qualified DNA sequence.

The genome database (GDB, http://www.gdb.org/) is a database of saving and process genomic map for human genome project (HGP). DDB constructs encyclopedia for human genome, besides constructing the genomic map, also develops a method of describing genome content of sequence level, comprising sequence variation and description with other functions and phenotype. GDB database saves data by an object model, providing service of searching a Web-based data object. A user may search various kinds of objects, and view the genomic map by means of graphics mode. For example, in UCSC database, DNA sequence in the sequence tagged site region of chromosome Y may be found based on the location coordinate of human genome reference sequence Hg19 (http://genome.ucsc.edu/). In specific examples, the location coordinate at which the DNA sequence in the sequence tagged site region of a chromosome was found in genome database, which was submitted to Roche-NimbleGen or other companies providing DNA capturing service. Designation and synthesis were completed by such companies.

Preferably, the apparatus of the present disclosure further comprises a mixture library constructing device, wherein the mixture library constructing device further comprises:

a plurality of DNA libraries constructing unit, for constructing a plurality of DNA libraries for a plurality of single DNA samples, wherein each of the plurality of DNA libraries has a different adaptor respectively;
a mixing unit, for mixing the plurality of DNA libraries with a predetermined ratio; and
a mixture library constructing unit, for determining the resulting mixture as the mixture library, if the resulting mixture meets a requirement of quality control.

Preferably, the apparatus of the present disclosure further comprises: a quality control device, wherein the quality control device further comprises:

a first filtering unit, for filtering out first unqualified data in the sequencing data, to obtain first qualified sequencing data;
a sequencing depth value calculating unit, for aligning the first qualified sequencing to a reference genome sequence by short-sequence alignment software, and calculating a first correlation parameter of the sequencing depth value of each of the plurality of DNA samples and a second correlation parameter of the sequencing depth value for a given sequence tagged site region among the plurality of DNA samples;
a second filtering unit, for filtering out second unqualified sequencing data based on the first calculated correlation parameter, to obtain second qualified sequencing data; and
a third filtering unit, for filtering out third unqualified sequencing data based on the second calculated correlation parameter, to obtain third qualified sequencing data of the given sequence tagged site region.

Preferably, the micro-deletion result obtaining device further comprises:

a sequencing depth value normalizing unit, for subjecting a sequencing depth value of the sequence tagged site region to normalization, to obtain a normalized sequencing depth value; and
a micro-deletion result obtaining unit, for detecting an outlier of the sequencing depth value of the sequence tagged site region based on the normalized sequencing depth value using the mathematical statistics method, to obtain the result showing the presence or the absence of the micro-deletion in the sequence tagged site region of the chromosome in each of the plurality of DNA samples.

Preferably, the sequencing depth value normalizing unit is specifically used in dividing all of the sequencing depth values of each of the plurality of DNA samples for a same region by an average depth value of each of the plurality of DNA samples, to obtain the normalized depth values of the plurality of DNA samples for the same region.

Preferably, as shown in Fig.26, the the micro-deletion result obtaining unit further comprises:

a mean value and variance obtaining module 2601, for calculating a mean value and a variance based on all normalized sequencing depth values of each of the plurality of DNA samples for a same region;
a normal distribution curve obtaining module 2602, for obtaining a normal distribution curve with all of non-outliers for the same region, based on the calculated mean value and variance of all normalized depth values of each of the plurality of DNA samples for the same region;
a probability calculating module 2603, for calculating a probability for each of the plurality of DNA samples in every region with a specific sequencing depth value, based on the normal distribution curve; and
a first determining module 2604, for determining a first probability cut-off value based on the probability for each of the plurality of DNA samples in a given region with the specific sequencing depth value, and obtaining a result R1 showing the presence of the micro-deletion in the given region, if the probability for one of the plurality of DNA samples in the given region with the specific sequencing depth value is smaller than the first probability cut-off value.

In a preferred embodiment, shown in Fig.27, the the micro-deletion obtaining unit still further comprises:

a probability cut-off value determining module 2701, for subjecting the result R1 to experimental verification, to obtain a verification result, and determining a second probability cut-off value based on the verification result, wherein the second probability cut-off value is smaller than the first probability cut-off value; and

a second determining module 2702, for obtaining a result R2 showing the presence of the micro-deletion in the given region, if the probability for one of the plurality of DNA samples in the given region with the specific depth value is smaller than the second probability cut-off value.

As shown in Fig.28, in another preferred embodiment, the the micro-deletion obtaining unit still further comprises:

a first ratio D/S obtaining module 2801, for calculating a first ratio D/S between the normalized depth value of the plurality of DNA samples for a given region and a median of all sequencing depth values of each of the plurality of DNA samples, based on the normalized depth value of the sequence tagged site region of the plurality of the plurality of DNA samples;
a first ratio D/R obtaining module 2802, for calculating a first ratio D/R between the normalized depth value of the plurality of DNA samples for the given region and a median of the sequencing depth values of all regions, based on the normalized depth value of the sequence tagged site region of the plurality of DNA samples;
a first ratio cut-off value obtaining module 2803, for training a first ratio cut-off value with the first ratio D/S using ID3 algorithm, and training a second ratio cut-off value with the first ratio D/R using ID3 algorithm;
a third determining module 2804, for obtaining the result showing the absence of the micro-deletion in the given region of the plurality of DNA samples, if the first ratio D/S is greater than the first ratio cut-off value;
a fourth determining module 2805, for obtaining the result showing the absence of the micro-deletion in the given region of the plurality of DNA samples, if the first ratio D/S is smaller than the first ratio cut-off value and the first ratio D/R is greater than the second ratio cut-off value; and
a fifth determining module 2806, for obtaining the result showing the presence of the micro-deletion in the given region of the plurality of DNA samples, if the first ratio D/S is smaller than the first ratio cut-off value and the first ratio D/R is smaller than the second ratio cut-off value.

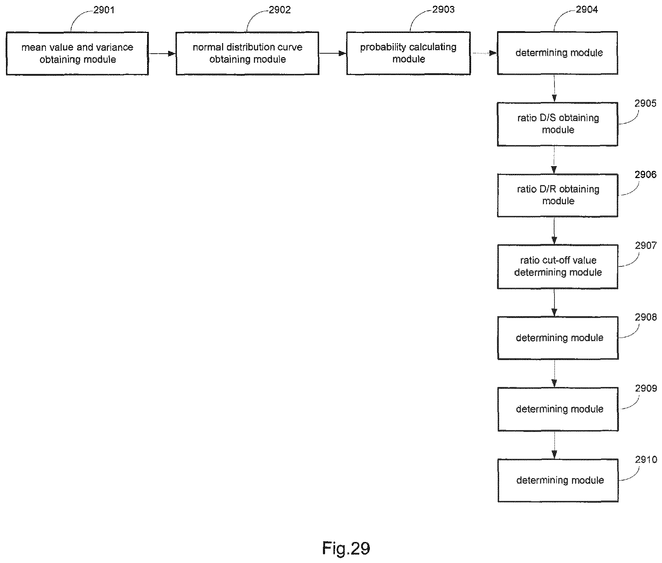

As shown in Fig.29, in a further preferred embodiment, the micro-deletion obtaining unit still further comprises:

a mean value and variance obtaining module 2901, for calculating a mean value and a variance based on all normalized sequencing depth values of each of the plurality of DNA samples for a same region;
a normal distribution curve obtaining module 2902, for obtaining a normal distribution curve with all of non-outliers for the same region, based on the calculated mean value and variance of all normalized depth values of each of the plurality of DNA samples for the same region;
a probability calculating module 2903, for calculating a probability for each of the plurality of DNA samples in every region with a specific sequencing depth value, based on the normal distribution curve;
a sixth determining module 2904, for determining a third probability cut-off value based on the probability for each of the plurality of DNA samples in a given region with the specific sequencing depth value, and obtaining a result R3 showing the presence of the micro-deletion in the given region, if the probability for one of the plurality of DNA samples in the given region with the specific sequencing depth value is smaller than the third probability cut-off value;
a second ratio D/S obtaining module 2905, for calculating a second ratio D/S between the normalized depth value of the plurality of DNA samples for the given region and a median of all sequencing depth values of each of the plurality of DNA samples in the result R3;
a second ratio D/R obtaining module 2906, for calculating a second ratio D/R between the normalized depth value of the plurality of DNA samples for the given region and a median of the sequencing depth values of all regions in the result R3;
a second ratio cut-off value determining module 2907, for training a third ratio cut-off value with the second ratio D/S using ID3 algorithm, and training a fourth ratio cut-off value with the second ratio D/R using ID3 algorithm;
a seventh determining module 2908, for obtaining the result showing the absence of the micro-deletion in the given region of the plurality of DNA samples, if the second ratio D/S is greater than the third ratio cut-off value;
an eighth determining module 2909, for obtaining the result showing the absence of the micro-deletion in the given region of the plurality of DNA samples, if the second ratio D/S is smaller than the third ratio cut-off value and the second ratio D/R is greater than the fourth ratio cut-off value; and
a ninth determining module 2910, for obtaining the result showing the presence of the micro-deletion in the given region of the plurality of DNA samples, if the second ratio D/S is smaller than the third ratio cut-off value and the second ratio D/R in the result R3 is smaller than the fourth ratio cut-off value.

The apparatus of detecting the micro-deletion in the sequence tagged site region of the chromosome and the apparatus thereof, by which obtains the capture probe covering an entire sequence tagged site region of a chromosome and captures DNA sequence of the sequence tagged site region from a plurality of DNA samples after being hybridized with a mixture library of the plurality of DNA samples, may efficiently and accurately detect reported or unreported micro-deletions in a sequence tagged site-related region of a chromosome with a large amount of DNA samples; besides the mathematical statistics analysis method used by the present disclosure may base on a normal distribution, an analysis method of decision tree, or a combination thereof followed by an experimental verification, which is scientific, stable, with a high sensitivity, a low false positive rate, being used in effective analysis for the micro-deletions.

Although explanatory embodiments have been shown and described, it would be appreciated by those skilled in the art that the above embodiments cannot be construed to limit the present disclosure, and changes, alternatives, and modifications can be made in the embodiments without departing from spirit, principles and scope of the present disclosure.

In the following, preferred embodiments are described to facilitate a deeper understanding of the invention:

1. A method of detecting a micro-deletion in a sequence tagged site region of a chromosome, comprising:

obtaining a capture probe corresponding to DNA sequence of the sequence tagged site region;
hybridizing the capture probe with a library of a plurality of DNA samples, to capture the DNA sequence of the sequence tagged site region from the plurality of DNA samples;
sequencing the captured DNA sequence of the sequence tagged site region to obtain sequencing data; and
analyzing the sequencing data using a mathematical statistics method, to obtain a result showing a presence or an absence of the micro-deletion in the sequence tagged site region of the chromosome in each of the plurality of DNA samples.

2. The method of embodiment 1, wherein the step of analyzing the sequencing data using the mathematical statistics test further comprises:

subjecting a sequencing depth value of the sequence tagged site region to normalization, to obtain a normalized sequencing depth value;
detecting an outlier of the sequencing depth value of the sequence tagged site region based on the normalized sequencing depth value using the mathematical statistics method, to obtain the result showing the presence or the absence of the micro-deletion in the sequence tagged site region of the chromosome in each of the plurality of DNA samples.

3. The method of embodiment 2, wherein the step of subjecting the sequencing depth value of the sequence tagged site region to normalization further comprises:
dividing all of the sequencing depth values of each of the plurality of DNA samples for a same region by an average depth value of each of the plurality of DNA samples, to obtain the normalized depth values of the plurality of DNA samples for the same region.

4. The method of embodiment 2, wherein the step of detecting the outlier of the sequencing depth value of the sequence tagged site region further comprises:

calculating a mean value and a variance based on all normalized sequencing depth values of each of the plurality of DNA samples for a same region;
obtaining a normal distribution curve with all of non-outliers for the same region, based on the calculated mean value and variance of all normalized depth values of each of the plurality of DNA samples for the same region;
calculating a probability for each of the plurality of DNA samples in every region with a specific sequencing depth value, based on the normal distribution curve;
determining a first probability cut-off value based on the probability for each of the plurality of DNA samples in a given region with the specific sequencing depth value; and
obtaining a result R1 showing the presence of the micro-deletion in the given region, if the probability for one of the plurality of DNA samples in the given region with the specific sequencing depth value is smaller than the first probability cut-off value.

5. The method of embodiment 4, wherein after the step of obtaining the result R1, the method further comprises:
subjecting the result R1 to experimental verification, to obtain a verification result;

determining a second probability cut-off value based on the verification result, wherein the second probability cut-off value is smaller than the first probability cut-off value;
obtaining a result R2 showing the presence of the micro-deletion in the given region, if the probability for one of the plurality of DNA samples in the given region with the specific depth value is smaller than the second probability cut-off value.

6. The method of embodiment 2, wherein the step of detecting the outlier of the sequencing depth value of the sequence tagged site region further comprises:

calculating a first ratio D/S between the normalized depth value of the plurality of DNA samples for a given region and a median of all sequencing depth values of each of the plurality of DNA samples, based on the normalized depth value of the sequence tagged site region of the plurality of the plurality of DNA samples;
calculating a first ratio D/R between the normalized depth value of the plurality of DNA samples for the given region and a median of the sequencing depth values of all regions, based on the normalized depth value of the sequence tagged site region of the plurality of DNA samples;
training a first ratio cut-off value with the first ratio D/S using ID3 algorithm, and training a second ratio cut-off value with the first ratio D/R using ID3 algorithm,
obtaining the result showing the absence of the micro-deletion in the given region of the plurality of DNA samples, if the first ratio D/S is greater than the first ratio cut-off value;
obtaining the result showing the absence of the micro-deletion in the given region of the plurality of DNA samples, if the first ratio D/S is smaller than the first ratio cut-off value and the first ratio D/R is greater than the second ratio cut-off value;
obtaining the result showing the presence of the micro-deletion in the given region of the plurality of DNA samples, if the first ratio D/S is smaller than the first ratio cut-off value and the first ratio D/R is smaller than the second ratio cut-off value.

7. The method of embodiment 2, wherein the step of detecting the outlier of the sequencing depth value of the sequence tagged site region further comprises:

calculating a mean value and a variance based on all normalized depth values of each of the plurality of DNA samples for a same region;
obtaining a normal distribution curve with all of non-outliers for the same region, based on the calculated mean value and variance of all normalized depth values of each of the plurality of DNA samples for the same region;
calculating a probability for each of the plurality of DNA samples in every region with a specific sequencing depth value, based on the normal distribution curve;
determining a third probability cut-off value based on the probability for each of the plurality of DNA samples in a given region with the specific sequencing depth value;
obtaining a result R3 showing the presence of the micro-deletion in the given region, if the probability for one of the plurality of DNA samples in the given region with the specific sequencing depth value is smaller than the third probability cut-off value;
calculating a second ratio D/S between the normalized depth value of the plurality of DNA samples for the given region and a median of all sequencing depth values of each of the plurality of DNA samples in the result R3;
calculating a second ratio D/R between the normalized depth value of the plurality of DNA samples for the given region and a median of the sequencing depth values of all regions in the result R3;
training a third ratio cut-off value with the second ratio D/S using ID3 algorithm, and training a fourth ratio cut-off value with the second ratio D/R using ID3 algorithm;
obtaining the result showing the absence of the micro-deletion in the given region of the plurality of DNA samples, if the second ratio D/S is greater than the third ratio cut-off value;
obtaining the result showing the absence of the micro-deletion in the given region of the plurality of DNA samples, if the second ratio D/S is smaller than the third ratio cut-off value and the second ratio D/R is greater than the fourth ratio cut-off value;
obtaining the result showing the presence of the micro-deletion in the given region of the plurality of DNA samples, if the second ratio D/S is smaller than the third ratio cut-off value and the second ratio D/R in the result R3 is smaller than the fourth ratio cut-off value.

8. The method of embodiment 1, wherein the step of obtaining the capture probe further comprises:

finding the DNA sequence of the sequence tagged site region within a genomic database;
selecting a qualified DNA sequence of the sequence tagged site region meeting requirement for designing the capture probe;
designing and synthesizing the capture probe based on the qualified DNA sequence.

9. The method of embodiment 1, wherein the mixture library of the plurality of DNA samples is constructed by following steps:

constructing a plurality of DNA libraries for a plurality of single DNA samples, wherein each of the plurality of DNA libraries has a different adaptor respectively;
mixing the plurality of DNA libraries with a predetermined ratio;
determining the resulting mixture as the mixture library, if the resulting mixture meets a requirement of quality control.

10. The method of embodiment 8, wherein in the step of constructing a plurality of DNA libraries for a plurality of single DNA samples, for each of the plurality of single DNA samples, the DNA library is constructed by following steps:

breaking a genome DNA into DNA fragments having a predetermined size by means of physical or chemical method, to obtain DNA fragments;
subjecting the DNA fragments to end-repairing, to obtain end-repaired DNA fragments with a phosphorylated end;
adding an "A" base to the end-repaired DNA fragments at 3'-end to obtain DNA fragments with the "A" base at 3'-end;
ligating an Index Adaptor to the DNA fragments with the "A" base at 3'-end, to obtain DNA fragments ligated with the Index Adaptor;
amplifying the DNA fragments ligated with the Index Adaptor using a primer comprising a sequence specific to the Index Adaptor, to obtain an amplification product; and
determining the amplification product as the DNA library, if the amplification product meets the requirement of quality control.

11. The method of embodiment 1, wherein after the step of sequencing the captured DNA sequencing and prior to the step of analyzing the sequencing data, the method further comprises:
subjecting the sequencing data to a quality control.

12. The method of embodiment 10, wherein the step of subjecting the sequencing data to the quality control further comprises:

filtering out first unqualified data in the sequencing data, to obtain first qualified sequencing data;
aligning the first qualified sequencing to a reference genome sequence by short-sequence alignment software, and calculating a first correlation parameter of the sequencing depth value of each of the plurality of DNA samples and a second correlation parameter of the sequencing depth value for a given sequence tagged site region among the plurality of DNA samples;
filtering out second unqualified sequencing data based on the first calculated correlation parameter, to obtain second qualified sequencing data;
filtering out third unqualified sequencing data based on the second calculated correlation parameter, to obtain third qualified sequencing data of the given sequence tagged site region.

13. The method of embodiment 11, wherein the step of filtering out the first unqualified data in the sequencing data, to obtain the first qualified sequencing data further comprises:

obtaining a first sequencing data set by filtering out unqualified data of which contains more than a predetermined proportion of bases having a low-quality from the sequencing data;
obtaining a second sequencing data set by filtering out unqualified data of which contains more than 10% undetermined bases from the first sequencing data set;
obtaining a third sequencing data set by filtering out unqualified data of which contains a sequencing adaptor sequence from the second sequencing data set after being aligned to a sequencing adaptor sequence library;
obtaining the first qualified sequencing data by filtering out unqualified data of which contains an exogenous sequence from the third sequencing data set after being aligned to all introduced exogenous sequences.

14. The method of embodiment 11, wherein the step of filtering out the second unqualified sequencing data further comprises:

obtaining a lower quartile Q1, an upper quartile Q3 and an interquartile IQR by Quartile function and ranking all sequencing depth values of the plurality of DNA samples in the first qualified sequencing data in an ascending order;

filtering out unqualified sequencing data of which has a sequencing depth value beyond a range of from Q1-1.5IQR to Q3+1.5IQR, to obtain the second qualified sequencing data.

15. The method of embodiment 11, wherein the step of filtering out the third unqualified sequencing data further comprises:

obtaining a lower quartile Q1, an upper quartile Q3 and an interquartile IQR by Quartile function and ranking all sequencing depth values of the given region among different DNA samples in the second qualified sequencing data in an ascending order;

filtering out unqualified sequencing data of which a median of the sequencing depth values of the given region among different DNA samples is zero or greater than Q3+1.5IQR, to obtain the third qualified sequencing data.

Reference example 16. An apparatus for detecting a micro-deletion in a sequence tagged site region of a chromosome, comprising:

a capture probe obtaining module, for obtaining a capture probe corresponding to DNA sequence of the sequence tagged site region;

a hybridizing module, for hybridizing the capture probe with a mixture library of a plurality of DNA samples, to capture the DNA sequence of the sequence tagged site region from the plurality of DNA samples;

a sequencing data obtaining module, for sequencing the captured DNA sequence of the sequence tagged site region to obtain sequencing data; and

a micro-deletion result obtaining module, for analyzing the sequencing data using a mathematical statistics method, to obtain a result showing a presence or an absence of the micro-deletion in the sequence tagged site region of the chromosome in each of the plurality of DNA samples.

Reference example 17. The apparatus of reference example 15, wherein the micro-deletion result obtaining module further comprises:

a sequencing depth value normalizing unit, for subjecting a sequencing depth value of the sequence tagged site region to normalization, to obtain a normalized sequencing depth value;

a micro-deletion result obtaining unit, for detecting an outlier of the sequencing depth value of the sequence tagged site region based on the normalized sequencing depth value using the mathematical statistics method, to obtain the result showing the presence or the absence of the micro-deletion in the sequence tagged site region of the chromosome in each of the plurality of DNA samples.

Reference example 18. The apparatus of reference example 16, wherein the sequencing depth value normalizing unit is used in dividing all of the sequencing depth values of each of the plurality of DNA samples for a same region by an average depth value of each of the plurality of DNA samples, to obtain the normalized depth values of the plurality of DNA samples for the same region.

Reference example 19. The apparatus of reference example 16, wherein the micro-deletion result obtaining unit further comprises:

a mean value and variance obtaining module, for calculating a mean value and a variance based on all normalized sequencing depth values of each of the plurality of DNA samples for a same region;

a normal distribution curve obtaining module, for obtaining a normal distribution curve with all of non-outliers for the same region, based on the calculated mean value and variance of all normalized depth values of each of the plurality of DNA samples for the same region;

a probability calculating module, for calculating a probability for each of the plurality of DNA samples in every region with a specific sequencing depth value, based on the normal distribution curve; and

a first determining module, for determining a first probability cut-off value based on the probability for each of the plurality of DNA samples in a given region with the specific sequencing depth value, and obtaining a result R1 showing the presence of the micro-deletion in the given region, if the probability for one of the plurality of DNA samples in the given region with the specific sequencing depth value is smaller than the first probability cut-off value.

Reference example 20. The apparatus of reference example 19, wherein the micro-deletion obtaining unit still further comprises:

> a probability cut-off value determining module, for subjecting the result R1 to experimental verification, to obtain a verification result, and determining a second probability cut-off value based on the verification result, wherein the second probability cut-off value is smaller than the first probability cut-off value;
> a second determining module, for obtaining a result R2 showing the presence of the micro-deletion in the given region, if the probability for one of the plurality of DNA samples in the given region with the specific depth value is smaller than the second probability cut-off value.

Reference example 21. The apparatus of reference example 16, wherein the micro-deletion obtaining unit still further comprises:

> a first ratio D/S obtaining module, for calculating a first ratio D/S between the normalized depth value of the plurality of DNA samples for a given region and a median of all sequencing depth values of each of the plurality of DNA samples, based on the normalized depth value of the sequence tagged site region of the plurality of the plurality of DNA samples;
> a first ratio D/R obtaining module, for calculating a first ratio D/R between the normalized depth value of the plurality of DNA samples for the given region and a median of the sequencing depth values of all regions, based on the normalized depth value of the sequence tagged site region of the plurality of DNA samples;
> a first ratio cut-off value obtaining module, for training a first ratio cut-off value with the first ratio D/S using ID3 algorithm, and training a second ratio cut-off value with the first ratio D/R using ID3 algorithm;
> a third determining module, for obtaining the result showing the absence of the micro-deletion in the given region of the plurality of DNA samples, if the first ratio D/S is greater than the first ratio cut-off value;
> a fourth determining module, for obtaining the result showing the absence of the micro-deletion in the given region of the plurality of DNA samples, if the first ratio D/S is smaller than the first ratio cut-off value and the first ratio D/R is greater than the second ratio cut-off value; and
> a fifth determining module, for obtaining the result showing the presence of the micro-deletion in the given region of the plurality of DNA samples, if the first ratio D/S is smaller than the first ratio cut-off value and the first ratio D/R is smaller than the second ratio cut-off value.

Reference example 22. The apparatus of reference example 16, wherein the micro-deletion obtaining unit still further comprises:

> a mean value and variance obtaining module, for calculating a mean value and a variance based on all normalized sequencing depth values of each of the plurality of DNA samples for a same region;
> a normal distribution curve obtaining module, for obtaining a normal distribution curve with all of non-outliers for the same region, based on the calculated mean value and variance of all normalized depth values of each of the plurality of DNA samples for the same region;
> a probability calculating module, for calculating a probability for each of the plurality of DNA samples in every region with a specific sequencing depth value, based on the normal distribution curve;
> a sixth determining module, for determining a third probability cut-off value based on the probability for each of the plurality of DNA samples in a given region with the specific sequencing depth value, and obtaining a result R3 showing the presence of the micro-deletion in the given region, if the probability for one of the plurality of DNA samples in the given region with the specific sequencing depth value is smaller than the third probability cut-off value;
> a second ratio D/S obtaining module, for calculating a second ratio D/S between the normalized depth value of the plurality of DNA samples for the given region and a median of all sequencing depth values of each of the plurality of DNA samples in the result R3;
> a second ratio D/R obtaining module, for calculating a second ratio D/R between the normalized depth value of the plurality of DNA samples for the given region and a median of the sequencing depth values of all regions in the result R3;
> a second ratio cut-off value determining module, for training a third ratio cut-off value with the second ratio D/S using ID3 algorithm, and training a fourth ratio cut-off value with the second ratio D/R using ID3 algorithm;
> a seventh determining module, for obtaining the result showing the absence of the micro-deletion in the given region of the plurality of DNA samples, if the second ratio D/S is greater than the third ratio cut-off value;
> an eighth determining module, for obtaining the result showing the absence of the micro-deletion in the given region of the plurality of DNA samples, if the second ratio D/S is smaller than the third ratio cut-off value and the second ratio D/R is greater than the fourth ratio cut-off value;
a ninth determining module, for obtaining the result showing the presence of the micro-deletion in the given region of the plurality of DNA samples, if the second ratio D/S is smaller than the third ratio cut-off value and the second ratio D/R in the result R3 is smaller than the fourth ratio cut-off value.

Reference example 23. The apparatus of reference example 16, wherein the capture probe obtaining module further comprises:

a region finding unit, for finding the DNA sequence of the sequence tagged site region within a genomic database;
a sequence selecting unit, for selecting a qualified DNA sequence of the sequence tagged site region meeting requirement for designing the capture probe; and
a capture probe obtaining unit, for designing and synthesizing the capture probe based on the qualified DNA sequence.

Reference example 24. The apparatus of reference example 16, further comprising a mixture library constructing module, wherein the mixture library constructing module further comprises:

a plurality of DNA libraries constructing unit, for constructing a plurality of DNA libraries for a plurality of single DNA samples, wherein each of the plurality of DNA libraries has a different adaptor respectively;
a mixing unit, for mixing the plurality of DNA libraries with a predetermined ratio; and
a mixture library constructing unit, for determining the resulting mixture as the mixture library, if the resulting mixture meets a requirement of quality control.

Reference example 25. The apparatus of reference example 15, further comprising a quality control module, wherein the quality control module further comprises:

a first filtering unit, for filtering out first unqualified data in the sequencing data, to obtain first qualified sequencing data;
a sequencing depth value calculating unit, for aligning the first qualified sequencing to a reference genome sequence by short-sequence alignment software, and calculating a first correlation parameter of the sequencing depth value of each of the plurality of DNA samples and a second correlation parameter of the sequencing depth value for a given sequence tagged site region among the plurality of DNA samples;
a second filtering unit, for filtering out second unqualified sequencing data based on the first calculated correlation parameter, to obtain second qualified sequencing data; and
a third filtering unit, for filtering out third unqualified sequencing data based on the second calculated correlation parameter, to obtain third qualified sequencing data of the given sequence tagged site region.

26. A computer readable medium comprising an order, configured to perform the method according to any one of embodiments 1 to 15 by a processer.

## Claims

1. A method of detecting a micro-defetion in a sequence tagged site region of a chromosome, comprising:

obtaining a capture probe corresponding to the DNA sequence of the sequence tagged site region;
hybridizing the capture probe with a library of a plurality of DNA samples, to capture the DNA sequence of the sequence tagged site region from the plurality of DNA samples;
sequencing the captured DNA sequence of the sequence tagged site region to obtain sequencing data; and
analyzing the sequencing data using a mathematical statistics method, to obtain a result showing a presence or an absence of the micro-deletion in the sequence tagged site region of the chromosome in each of the plurality of DNA samples.

2. The method of claim 1, wherein the step of analyzing the sequencing data using the mathematical statistics test further comprises:

subjecting a sequencing depth value of the sequence tagged site region to normalization, to obtain a normalized sequencing depth value;

detecting an outlier of the sequencing depth value of the sequence tagged site region based on the normalized sequencing depth value using the mathematical statistics method, to obtain the result showing the presence or the absence of the micro-deletion in the sequence tagged site region of the chromosome in each of the plurality of DNA samples.

3. The method of claim 2, wherein the step of subjecting the sequencing depth value of the sequence tagged site region to normalization further comprises:
dividing all of the sequencing depth values of each of the plurality of DNA samples for a same region by an average depth value of each of the plurality of DNA samples, to obtain the normalized depth values of the plurality of DNA samples for the same region.

4. The method of claim 2, wherein the step of detecting the outlier of the sequencing depth value of the sequence tagged site region further comprises:

calculating a mean value and a variance based on all normalized sequencing depth values of each of the plurality of DNA samples for a same region;
obtaining a normal distribution curve with all of non-outliers for the same region, based on the calculated mean value and variance of all normalized depth values of each of the plurality of DNA samples for the same region;
calculating a probability for each of the plurality of DNA samples in every region with a specific sequencing depth value, based on the normal distribution curve;
determining a first probability cut-off value based on the probability for each of the plurality of DNA samples in a given region with the specific sequencing depth value;
obtaining a result R1 showing the presence of the micro-deletion in the given region, if the probability for one of the plurality of DNA samples in the given region with the specific sequencing depth value is smaller than the first probability cut-off value,
wherein optionally, after the step of obtaining the result R1, the method further comprises:

subjecting the result R1 to experimental verification, to obtain a verification result;
determining a second probability cut-off value based on the verification result, wherein the second probability cut-off value is smaller than the first probability cut-off value; and
obtaining a result R2 showing the presence of the micro-deletion in the given region, if the probability for one of the plurality of DNA samples in the given region with the specific depth value is smaller than the second probability cut-off value.

5. The method of claim 2, wherein the step of detecting the outlier of the sequencing depth value of the sequence tagged site region further comprises:

calculating a first ratio D/S between the normalized depth value of the plurality of DNA samples for a given region and a median of all sequencing depth values of each of the plurality of DNA samples, based on the normalized depth value of the sequence tagged site region of the plurality of the plurality of DNA samples;
calculating a first ratio D/R between the normalized depth value of the plurality of DNA samples for the given region and a median of the sequencing depth values of all regions, based on the normalized depth value of the sequence tagged site region of the plurality of DNA samples;
training a first ratio cut-off value with the first ratio D/S using ID3 algorithm, and training a second ratio cut-off value with the first ratio D/R using ID3 algorithm,
obtaining the result showing the absence of the micro-deletion in the given region of the plurality of DNA samples, if the first ratio D/S is greater than the first ratio cut-off value;
obtaining the result showing the absence of the micro-deletion in the given region of the plurality of DNA samples, if the first ratio D/S is smaller than the first ratio cut-off value and the first ratio D/R is greater than the second ratio cut-off value;
obtaining the result showing the presence of the micro-deletion in the given region of the plurality of DNA samples, if the first ratio D/S is smaller than the first ratio cut-off value and the first ratio D/R is smaller than the second ratio cut-off value.

6. The method of claim 2, wherein the step of detecting the outlier of the sequencing depth value of the sequence tagged site region further comprises:

calculating a mean value and a variance based on all normalized depth values of each of the plurality of DNA samples for a same region;

obtaining a normal distribution curve with all of non-outliers for the same region, based on the calculated mean value and variance of all normalized depth values of each of the plurality of DNA samples for the same region;

calculating a probability for each of the plurality of DNA samples in every region with a specific sequencing depth value, based on the normal distribution curve;

determining a third probability cut-off value based on the probability for each of the plurality of DNA samples in a given region with the specific sequencing depth value;

obtaining a result R3 showing the presence of the micro-deletion in the given region, if the probability for one of the plurality of DNA samples in the given region with the specific sequencing depth value is smaller than the third probability cut-off value;

calculating a second ratio D/S between the normalized depth value of the plurality of DNA samples for the given region and a median of all sequencing depth values of each of the plurality of DNA samples in the result R3;

calculating a second ratio D/R between the normalized depth value of the plurality of DNA samples for the given region and a median of the sequencing depth values of all regions in the result R3;

training a third ratio cut-off value with the second ratio D/S using ID3 algorithm, and training a fourth ratio cut-off value with the second ratio D/R using ID3 algorithm;

obtaining the result showing the absence of the micro-deletion in the given region of the plurality of DNA samples, if the second ratio D/S is greater than the third ratio cut-off value;

obtaining the result showing the absence of the micro-deletion in the given region of the plurality of DNA samples, if the second ratio D/S is smaller than the third ratio cut-off value and the second ratio D/R is greater than the fourth ratio cut-off value;

obtaining the result showing the presence of the micro-deletion in the given region of the plurality of DNA samples, if the second ratio D/S is smaller than the third ratio cut-off value and the second ratio D/R in the result R3 is smaller than the fourth ratio cut-off value.

7. The method of claim 1, wherein after the step of sequencing the captured DNA sequencing and prior to the step of analyzing the sequencing data, the method further comprises:

subjecting the sequencing data to a quality control,

wherein optionally, the step of subjecting the sequencing data to the quality control further comprises:

filtering out first unqualified data in the sequencing data, to obtain first qualified sequencing data;

aligning the first qualified sequencing to a reference genome sequence by short-sequence alignment software, and calculating a first correlation parameter of the sequencing depth value of each of the plurality of DNA samples and a second correlation parameter of the sequencing depth value for a given sequence tagged site region among the plurality of DNA samples;

filtering out second unqualified sequencing data based on the first calculated correlation parameter, to obtain second qualified sequencing data;

filtering out third unqualified sequencing data based on the second calculated correlation parameter, to obtain third qualified sequencing data of the given sequence tagged site region,

wherein optionally, the step of filtering out the second unqualified sequencing data further comprises:

obtaining a lower quartile Q1, an upper quartile Q3 and an interquartile IQR by Quartile function and ranking all sequencing depth values of the plurality of DNA samples in the first qualified sequencing data in an ascending order;

filtering out unqualified sequencing data of which has a sequencing depth value beyond a range of from Q1-1.5IQR to Q3+1.51QR, to obtain the second qualified sequencing data,

wherein optionally, the step of filtering out the third unqualified sequencing data further comprises:

obtaining a lower quartile Q1, an upper quartile Q3 and an interquartile IQR by Quartile function and ranking all sequencing depth values of the given region among different DNA samples in the second qualified sequencing data in an ascending order;

filtering out unqualified sequencing data of which a median of the sequencing depth values of the given region among different DNA samples is zero or greater than Q3+1.5IQR, to obtain the third qualified sequencing data.

8.  A computer readable medium comprising instructions configured to perform the method according to any one of claims 1 to 7 by a processor.

**Patentansprüche**

1.  Verfahren zur Detektion von Mikrodelition in eines sequence-tagged-site-Bereichs eines Chromosoms, umfassend:

    Erhalten einer Fangsonde, welche zu der DNA-Sequenz des sequence-tagged-site-Bereichs entspricht,
    Hybridisieren der Fangsonde mit einer Sammlung einer vielzahl von DNA-Proben, um die DNA-Sequenz des sequence-tagged-site-Bereichs aus der Vielzahl der DNA-Proben einzufangen,
    Sequenzieren der eingefangenen DNA-Sequenz des sequence-tagged-site-Bereichs, um Sequenzierungsdaten zu erhalten,
    Analysieren der Sequenzierungsdaten unter Verwendung einer mathematischen Statistik-Methode, um ein Ergebnis zu erhalten, welches bei jeder der Vielzahl der DNA-Proben ein Vorliegen oder ein Nicht-Vorliegen der Mikrodelition in dem sequence-tagged-site-Bereich des Chromosoms zeigt.

2.  Verfahren nach Anspruch 1, wobei der Schritt des Analysierens der Sequenzierungsdaten unter Verwendung des mathematischen Statistiktests weiterhin umfasst:

    Normieren eines Sequenzierungstiefenwertes des sequence-tagged-site-Bereichs, um einen normierten Sequenzierungstiefenwert zu erhalten;
    Erkennen eines Ausreißers des Sequenzierungstiefenwertes des sequence-tagged-site-Bereichs basierend auf der dem normierten Sequenzierungstiefenwert unter Verwendung der mathematischen Statistik-Methode, um das Ergebnis zu erhalten, welches bei jeder der Vielzahl der DNA-Proben das Vorliegen oder das Nicht-Vorliegen der Mikrodelition in dem sequence-tagged-site-Bereich des Chromosoms zeigt.

3.  Verfahren nach Anspruch 2, wobei der Schritt des Normierens des Sequenzierungstiefenwertes des sequence-tagged-site-Bereichs weiterhin umfasst:
    Teilen aller Sequenzierungstiefenwerte jeder der Vielzahl der DNA-Proben für einen gleichen Bereich durch einen mittleren Tiefenwert jeder der DNA-Proben, um den normierten Tiefenwert der Vielzahl der DNA-Proben für den gleichen Bereich zu erhalten.

4.  Verfahren nach Anspruch 2, wobei der Schritt des Erkennens des Ausreißers des Sequenzierungstiefenwertes des sequence-tagged-site-Bereichs weiterhin umfassst:

    Berechnen eines mittleren Werts und einer Varianz basierend auf allen normierten Sequenzierungstiefenwerten jeder der Vielzahl der DNA-Proben für einen gleichen Bereich;
    Erhalten einer Normalverteilungskurve mit allen Nichtausreißern für den gleichen Bereich basierend auf dem berechneten mittleren Wert und der berechneten Varianz aller normierten Tiefenwerte jeder der Vielzahl der DNA-Proben des gleichen Bereichs;
    Berechnen einer Wahrscheinlichkeit für jede der Vielzahl der DNA-Proben in jedem Bereich mit einem spezifischen Sequenzierungstiefenwert basierend auf der Normalverteilungskurve;
    Bestimmen eines ersten Wahrscheinlichkeits-Grenzwertes basierend auf der Wahrscheinlichkeit für jede der Vielzahl der DNA-Proben in einem vorgegebenen Bereich mit dem spezifischen Sequenzierungstiefenwert;
    Erhalten eines Ergebnisses R1, welches das Vorliegen der Mikrodelition in dem vorgegebenen Bereich zeigt, wenn die Wahrscheinlichkeit für eine der Vielzahl der DNA-Proben in dem vorgegebenen Bereich mit dem spezifischen Sequenzierungstiefenwert kleiner ist als der erste Wahrscheinlichkeits-Grenzwert,
    wobei das Verfahren optional nach dem Schritt des Erhaltens des Ergebnisses R1 weiterhin umfasst:
    Experimentelles Verifizieren des Ergebnisses R1, um ein Verifikationsergebnis zu erhalten;
    Bestimmen eines zweiten Wahrscheinlichkeits-Grenzwerts basierend auf dem Verifikations-Ergebnis, wobei der zweite Wahrscheinlichkeits-Grenzwert kleiner ist als der erste Wahrscheinlichkeits-Grenzwert; und
    Erhalten eines Ergebnisses R2, welches das Vorliegen der Mikrodelition in dem vorgegebenen Bereich zeigt, wenn die Wahrscheinlichkeit für eine der Vielzahl der DNA-Proben in dem vorgegebenen Bereich mit dem spezifischen Sequenzierungstiefenwert kleiner ist als der zweite Wahrscheinlichkeits-Grenzwert,

5.  Verfahren nach Anspruch 2, wobei der Schritt des Erkennens des Ausreißers des Sequenzierungstiefenwertes des sequence-tagged-site-Bereichs weiterhin umfassst:

Berechnen eines ersten Verhältnisses D/S zwischen dem normierten Tiefenwert der Vielzahl der DNA-Proben für einen vorgegebenen Bereich und eines Medians für alle Sequenzierungstiefenwerte für jede der Vielzahl der DNA-Proben basierend auf dem normierten Tiefenwert des sequence-tagged-site-Bereichs der Vielzahl der Vielzahl der DNA-Proben;

Berechnen eines ersten Verhältnisses D/R zwischen dem normierten Tiefenwert der Vielzahl der DNA-Proben für den vorgegebenen Bereich und eines Medians der Sequenzierungstiefenwerte aller Bereiche basierend auf dem normierten Tiefenwert des sequence-tagged-site-Bereichs der Vielzahl der DNA-Proben;

Lernen eines ersten Grenzwertes mit dem ersten Verhältnis D/S unter Verwendung eines ID3-Algorithmus und Lernen eines zweiten Grenzwertes mit dem ersten Verhältnis D/R unter Verwendung eines ID3-Algorithmus,

Erhalten des Ergebnisses, welches das Nicht-Vorliegen der Mikrodelition in dem vorgegebenen Bereich der Vielzahl der DNA-Proben zeigt, wenn das erste Verhältnis D/S größer ist als der erste Grenzwert;

Erhalten des Ergebnisses, welches das Nicht-Vorliegen der Mikrodelition in dem vorgegebenen Bereich der Vielzahl der DNA-Proben zeigt, wenn das erste Verhältnis D/S kleiner als der erste Grenzwert ist und das erste Verhältnis D/R größer als der zweite Grenzwert ist;

Erhalten des Ergebnisses, welches das Vorliegen der Mikrodelition in dem vorgegebenen Bereich der Vielzahl der DNA-Proben zeigt, wenn das erste Verhältnis D/S kleiner als der erste Grenzwert ist und das erste Verhältnis D/R kleiner als der zweite Grenzwert ist.

6. Verfahren nach Anspruch 2, wobei der Schritt des Erkennens des Ausreißers des Sequenzierungstiefenwertes des sequence-tagged-site-Bereichs weiterhin umfassst:

Berechnen eines mittleren Werts und einer Varianz basierend auf allen normierten Sequenzierungstiefenwerten jeder der Vielzahl der DNA-Proben für einen gleichen Bereich;

Erhalten einer Normalverteilungskurve mit allen Nichtausreißern für den gleichen Bereich basierend auf dem berechneten mittleren Wert und der berechneten Varianz aller normierten Tiefenwerte jeder der Vielzahl der DNA-Proben des gleichen Bereichs;

Berechnen einer Wahrscheinlichkeit für jede der Vielzahl der DNA-Proben in jedem Bereich mit einem spezifischen Sequenzierungstiefenwert basierend auf der Normalverteilungskurve;

Bestimmen eines dritten Wahrscheinlichkeits-Grenzwertes basierend auf der Wahrscheinlichkeit für jede der Vielzahl der DNA-Proben in einem vorgegebenen Bereich mit dem spezifischen Sequenzierungstiefenwert;

Erhalten eines Ergebnisses R3, welches das Vorliegen der Mikrodelition in dem vorgegebenen Bereich zeigt, wenn die Wahrscheinlichkeit für eine der Vielzahl der DNA-Proben in dem vorgegebenen Bereich mit dem spezifischen Sequenzierungstiefenwert kleiner ist als der dritte Wahrscheinlichkeits-Grenzwert;

Berechnen eines zweiten Verhältnisses D/S zwischen dem normierten Tiefenwert der Vielzahl der DNA-Proben für den vorgegebenen Bereich und eines Medians für alle Sequenzierungstiefenwerte für jede der Vielzahl der DNA-Proben in dem Ergebnis R3;

Berechnen eines zweiten Verhältnisses D/R zwischen dem normierten Tiefenwert der Vielzahl der DNA-Proben für den vorgegebenen Bereich und eines Medians der Sequenzierungstiefenwerte aller Bereiche in dem Ergebnis R3;

Lernen eines dritten Grenzwertes mit dem zweiten Verhältnis D/S unter Verwendung eines ID3-Algorithmus und Lernen eines vierten Grenzwertes mit dem zweiten Verhältnis D/R unter Verwendung eines ID3-Algorithmus,

Erhalten des Ergebnisses, welches das Nicht-Vorliegen der Mikrodelition in dem vorgegebenen Bereich der Vielzahl der DNA-Proben zeigt, wenn das zweite Verhältnis D/S größer als der dritte Grenzwert;

Erhalten des Ergebnisses, welches das Nicht-Vorliegen der Mikrodelition in dem vorgegebenen Bereich der Vielzahl der DNA-Proben zeigt, wenn das zweite Verhältnis D/S kleiner als der dritte Grenzwert ist und das zweite Verhältnis D/S größer als der vierte Grenzwert ist;

Erhalten des Ergebnisses, welches das Vorliegen der Mikrodelition in dem vorgegebenen Bereich der Vielzahl der DNA-Proben zeigt, wenn das zweite Verhältnis D/S kleiner als der dritte Grenzwert ist und das zweite Verhältnis D/R in dem Ergebnis R3 kleiner als der vierte Grenzwert ist.

7. Verfahren nach Anspruch 1, wobei das Verfahren nach dem Schritt des Sequenzierens der eingefangenen DNA-Sequenz und vor dem Schritt des Analysierens der Sequenzierungsdaten weiterhin umfasst:

Qualitätskontrolle der Sequenzierungsdaten,
wobei der Schritt der Qualitätskontrolle der Sequenzierungsdaten optional umfasst:
Herausfiltern erster unqualifizierter Daten in den Sequenzierungsdaten, um erste qualifizierte Sequenzierungsdaten zu erhalten;
Abgleichen der ersten qualifizierten Sequenzierungsdaten an einem Referenzgenom durch eine Kurzsequenz-Abgleich-Software und Berechnen eines ersten Korrelationsparameters des Sequenzierungstiefenwertes jeder der Vielzahl der DNA-Proben und eines zweiten Korrelationsparameters des Sequenzierungstiefenwertes für einen vorgegebenen sequence-tagged-site-Bereich unter der Vielzahl der DNA-Proben;

Herausfiltern zweiter unqualifizierter Sequenzierungsdaten basierend auf dem ersten berechneten Korrelationsparameter, um zweite qualifizierte Sequenzierungsdaten zu erhalten;

Herausfiltern dritter unqualifizierter Sequenzierungsdaten basierend auf dem zweiten berechneten Korrelationsparameter, um dritte qualifizierte Sequenzierungsdaten des vorgegebenen sequence-tagged-site-Bereichs zu erhalten,

wobei der Schritt des Herausfilterns der zweiten unqualifizierten Sequenzierungsdaten optional weiterhin umfasst:

Erhalten eines unteren Quartils Q1, eines oberen Quartils Q3 und eines Interquartilsabstandes IQR durch Quartilsfunktion und Ordnung aller Sequenzierungstiefenwerte der Vielzahl der DNA-Proben in den ersten qualifizierten Sequenzierungsdaten in aufsteigender Reihenfolge;

Herausfiltern unqualifizierter Sequenzierungsdaten, von welchen ein Sequenzierungstiefenwert außerhalb des Bereichs von Q1 - 1,5 IQR bis Q3 + 1,5 IQR liegt, um die zweiten qualifizierten Sequenzierungsdaten zu erhalten,

wobei der Schritt des Herausfilterns der dritten unqualifizierten Sequenzierungsdaten optional weiterhin umfasst:

Erhalten eines unteren Quartils Q1, eines oberen Quartils Q3 und eines Interquartilsabstandes IQR durch Quartilsfunktion und Ordnung aller Sequenzierungstiefenwerte des vorgegebenen Bereichs in den zweiten qualifizierten Sequenzierungsdaten in aufsteigender Reihenfolge;

Herausfiltern unqualifizierter Sequenzierungsdaten, von welchen ein Median der Sequenzierungstiefenwerte des vorgegebenen Bereichs unter verschiedenen DNA-Proben null oder größer als Q3 + 1,5 IQR ist, um die dritten qualifizierten Sequenzierungsdaten zu erhalten.

8. Computerlesbares Medium umfassend Instruktionen, die zur Ausführung des Verfahrens gemäß einem der Ansprüche 1 bis 7 durch einen Prozessor konfiguriert sind.

**Revendications**

1. Procédé de détection d'une microdélétion dans une région de site étiqueté par une séquence d'un chromosome, comprenant :

l'obtention d'une sonde de capture correspondant à la séquence d'ADN de la région de site étiqueté par une séquence ;

l'hybridation de la sonde de capture avec une banque d'une pluralité d'échantillons d'ADN, pour capturer la séquence d'ADN de la région de site étiqueté par une séquence à partir de la pluralité d'échantillons d'ADN ;

le séquençage de la séquence d'ADN capturée de la région de site étiqueté par une séquence pour obtenir des données de séquençage ; et

l'analyse des données de séquençage au moyen d'une méthode de statistique mathématique, pour obtenir un résultat montrant une présence ou une absence de la microdélétion dans la région de site étiqueté par une séquence du chromosome dans chacun de la pluralité d'échantillons d'ADN.

2. Procédé de la revendication 1, dans lequel l'étape d'analyse des données de séquençage au moyen du test de statistique mathématique comprend en outre :

la soumission d'une valeur de profondeur de séquençage de la région de site étiqueté par une séquence à une normalisation, pour obtenir une valeur de profondeur de séquençage normalisée ;

la détection d'un cas aberrant de la valeur de profondeur de séquençage de la région de site étiqueté par une séquence sur la base de la valeur de profondeur de séquençage normalisée au moyen de la méthode de statistique mathématique, pour obtenir le résultat montrant la présence ou l'absence de la microdélétion dans la région de site étiqueté par une séquence du chromosome dans chacun de la pluralité d'échantillons d'ADN.

3. Procédé de la revendication 2, dans lequel l'étape de soumission de la valeur de profondeur de séquençage de la région de site étiqueté par une séquence à une normalisation comprend en outre :

la division de toutes les valeurs de profondeur de séquençage de chacun de la pluralité d'échantillons d'ADN pour une même région par une valeur de profondeur moyenne de chacun de la pluralité d'échantillons d'ADN, pour obtenir les valeurs de profondeur normalisées de la pluralité d'échantillons d'ADN pour la même région.

**4.** Procédé de la revendication 2, dans lequel l'étape de détection du cas aberrant de la valeur de profondeur de séquençage de la région de site étiqueté par une séquence comprend en outre :

le calcul d'une valeur moyenne et d'une variance sur la base de toutes les valeurs de profondeur de séquençage normalisées de chacun de la pluralité d'échantillons d'ADN pour une même région ;

l'obtention d'une courbe de distribution normale avec tous les cas non aberrants pour la même région, sur la base de la valeur moyenne et de la variance calculées de toutes les valeurs de profondeur normalisées de chacun de la pluralité d'échantillons d'ADN pour la même région ;

le calcul d'une probabilité pour chacun de la pluralité d'échantillons d'ADN dans chaque région avec une valeur de profondeur de séquençage spécifique, sur la base de la courbe de distribution normale ;

la détermination d'une première valeur limite de probabilité sur la base de la probabilité pour chacun de la pluralité d'échantillons d'ADN dans une région donnée avec la valeur de profondeur de séquençage spécifique ;

l'obtention d'un résultat R1 montrant la présence de la microdélétion dans la région donnée, si la probabilité pour un de la pluralité d'échantillons d'ADN dans la région donnée avec la valeur de profondeur de séquençage spécifique est inférieure à la première valeur limite de probabilité,

dans lequel éventuellement, après l'étape d'obtention du résultat R1, le procédé comprend en outre :

la soumission du résultat R1 à une vérification expérimentale, pour obtenir un résultat de vérification ;

la détermination d'une deuxième valeur limite de probabilité sur la base du résultat de vérification, la deuxième valeur limite de probabilité étant inférieure à la première valeur limite de probabilité ; et

l'obtention d'un résultat R2 montrant la présence de la microdélétion dans la région donnée, si la probabilité pour un de la pluralité d'échantillons d'ADN dans la région donnée avec la valeur de profondeur spécifique est inférieure à la deuxième valeur limite de probabilité.

**5.** Procédé de la revendication 2, dans lequel l'étape de détection du cas aberrant de la valeur de profondeur de séquençage de la région de site étiqueté par une séquence comprend en outre :

le calcul d'un premier rapport D/S entre la valeur de profondeur normalisée de la pluralité d'échantillons d'ADN pour une région donnée et une médiane de toutes les valeurs de profondeur de séquençage de chacun de la pluralité d'échantillons d'ADN, sur la base de la valeur de profondeur normalisée de la région de site étiqueté par une séquence de la pluralité de la pluralité d'échantillons d'ADN ;

le calcul d'un premier rapport D/R entre la valeur de profondeur normalisée de la pluralité d'échantillons d'ADN pour la région donnée et une médiane des valeurs de profondeur de séquençage de toutes les régions, sur la base de la valeur de profondeur normalisée de la région de site étiqueté par une séquence de la pluralité d'échantillons d'ADN ;

l'apprentissage d'une première valeur limite de rapport avec le premier rapport D/S au moyen de l'algorithme ID3, et l'apprentissage d'une deuxième valeur limite de rapport avec le premier rapport D/R au moyen de l'algorithme ID3 ;

l'obtention du résultat montrant l'absence de la microdélétion dans la région donnée de la pluralité d'échantillons d'ADN, si le premier rapport D/S est supérieur à la première valeur limite de rapport ;

l'obtention du résultat montrant l'absence de la microdélétion dans la région donnée de la pluralité d'échantillons d'ADN, si le premier rapport D/S est inférieur à la première valeur limite de rapport et le premier rapport D/R est supérieur à la deuxième valeur limite de rapport ;

l'obtention du résultat montrant la présence de la microdélétion dans la région donnée de la pluralité d'échantillons d'ADN, si le premier rapport D/S est inférieur à la première valeur limite de rapport et le premier rapport D/R est inférieur à la deuxième valeur limite de rapport.

**6.** Procédé de la revendication 2, dans lequel l'étape de détection du cas aberrant de la valeur de profondeur de séquençage de la région de site étiqueté par une séquence comprend en outre :

le calcul d'une valeur moyenne et d'une variance sur la base de toutes les valeurs de profondeur normalisées de chacun de la pluralité d'échantillons d'ADN pour une même région ;

l'obtention d'une courbe de distribution normale avec tous les cas non aberrants pour la même région, sur la base de la valeur moyenne et de la variance calculées de toutes les valeurs de profondeur normalisées de chacun de la pluralité d'échantillons d'ADN pour la même région ;

le calcul d'une probabilité pour chacun de la pluralité d'échantillons d'ADN dans chaque région avec une valeur de profondeur de séquençage spécifique, sur la base de la courbe de distribution normale ;

la détermination d'une troisième valeur limite de probabilité sur la base de la probabilité pour chacun de la pluralité d'échantillons d'ADN dans une région donnée avec la valeur de profondeur de séquençage spécifique ;

l'obtention d'un résultat R3 montrant la présence de la microdélétion dans la région donnée, si la probabilité pour un de la pluralité d'échantillons d'ADN dans la région donnée avec la valeur de profondeur de séquençage spécifique est inférieure à la troisième valeur limite de probabilité ;

le calcul d'un deuxième rapport D/S entre la valeur de profondeur normalisée de la pluralité d'échantillons d'ADN pour la région donnée et une médiane de toutes les valeurs de profondeur de séquençage de chacun de la pluralité d'échantillons d'ADN dans le résultat R3 ;

le calcul d'un deuxième rapport D/R entre la valeur de profondeur normalisée de la pluralité d'échantillons d'ADN pour la région donnée et une médiane des valeurs de profondeur de séquençage de toutes les régions dans le résultat R3 ;

l'apprentissage d'une troisième valeur limite de rapport avec le deuxième rapport D/S au moyen de l'algorithme ID3, et l'apprentissage d'une quatrième valeur limite de rapport avec le deuxième rapport D/R au moyen de l'algorithme ID3 ;

l'obtention du résultat montrant l'absence de la microdélétion dans la région donnée de la pluralité d'échantillons d'ADN, si le deuxième rapport D/S est supérieur à la troisième valeur limite de rapport ;

l'obtention du résultat montrant l'absence de la microdélétion dans la région donnée de la pluralité d'échantillons d'ADN, si le deuxième rapport D/S est inférieur à la troisième valeur limite de rapport et le deuxième rapport D/R est supérieur à la quatrième valeur limite de rapport ;

l'obtention du résultat montrant la présence de la microdélétion dans la région donnée de la pluralité d'échantillons d'ADN, si le deuxième rapport D/S est inférieur à la troisième valeur limite de rapport et le deuxième rapport D/R dans le résultat R3 est inférieur à la quatrième valeur limite de rapport.

7. Procédé de la revendication 1, dans lequel après l'étape de séquençage du séquençage d'ADN capturé et avant l'étape d'analyse des données de séquençage, le procédé comprend en outre :

la soumission des données de séquençage à un contrôle de qualité,

dans lequel éventuellement, l'étape de soumission des données de séquençage au contrôle de qualité comprend en outre :

l'élimination par filtrage de premières données non qualifiées dans les données de séquençage, pour obtenir des premières données de séquençage qualifiées ;

l'alignement du premier séquençage qualifié avec une séquence de génome de référence par un logiciel d'alignement de séquences courtes, et le calcul d'un premier paramètre de corrélation de la valeur de profondeur de séquençage de chacun de la pluralité d'échantillons d'ADN et d'un deuxième paramètre de corrélation de la valeur de profondeur de séquençage pour une région de site étiqueté par une séquence donnée parmi la pluralité d'échantillons d'ADN ;

l'élimination par filtrage de deuxièmes données de séquençage non qualifiées sur la base du premier paramètre de corrélation calculé, pour obtenir des deuxièmes données de séquençage qualifiées ;

l'élimination par filtrage de troisièmes données de séquençage non qualifiées sur la base du deuxième paramètre de corrélation calculé, pour obtenir des troisièmes données de séquençage qualifiées de la région de site étiqueté par une séquence donnée,

dans lequel éventuellement, l'étape d'élimination par filtrage des deuxièmes données de séquençage non qualifiées comprend en outre :

l'obtention d'un quartile inférieur Q1, d'un quartile supérieur Q3 et d'un interquartile IQR par la fonction Quartile et le classement de toutes les valeurs de profondeur de séquençage de la pluralité d'échantillons d'ADN dans les premières données de séquençage qualifiées dans un ordre ascendant ;

l'élimination par filtrage de données de séquençage non qualifiées qui ont une valeur de profondeur de séquençage au-delà d'une gamme de Q1-1,5IQR à Q3+1,5IQR, pour obtenir les deuxièmes données de séquençage qualifiées,

dans lequel éventuellement, l'étape d'élimination par filtrage des troisièmes données de séquençage non qualifiées comprend en outre :

l'obtention d'un quartile inférieur Q1, d'un quartile supérieur Q3 et d'un interquartile IQR par la fonction Quartile et le classement de toutes les valeurs de profondeur de séquençage de la région donnée parmi différents échantillons d'ADN dans les deuxièmes données de séquençage qualifiées dans un ordre ascendant ;

l'élimination par filtrage de données de séquençage non qualifiées dont une médiane des valeurs de profondeur de séquençage de la région donnée parmi différents échantillons d'ADN est nulle ou supérieure à Q3+1,5IQR, pour obtenir les troisièmes données de séquençage qualifiées.

8. Support lisible par ordinateur comprenant une instruction configurée pour faire effectuer le procédé selon l'une quelconque des revendications 1 à 7 par un processeur.

obtaining a capture probe corresponding to DNA sequence
of the sequence tagged site region

101

hybridizing the capture probe with a mixture library of a plurality of DNA samples, to capture
the DNA sequence of the sequence tagged site region from the plurality of DNA samples

102

sequencing the captured DNA sequence of the sequence tagged site region
to obtain sequencing data

103

analyzing the sequencing data using a mathematical statistics method, to obtain a result
showing a presence or an absence of the micro-deletion in the sequence tagged site region
of the chromosome in each of the plurality of DNA samples

104

Fig.1

finding the DNA sequence of the sequence tagged site region
within a genomic database

201

selecting a qualified DNA sequence of the sequence tagged site
region meeting requirement for designing the capture probe

202

designing and synthesizing the capture probe
based on the qualified DNA sequence

203

Fig.2

constructing a plurality of DNA libraries for a plurality of single DNA samples, wherein
each of the plurality of DNA libraries has a different adaptor respectively

301

mixing the plurality of DNA libraries with a predetermined ratio

302

determining the resulting mixture as the mixture library,
if the resulting mixture meets a requirement of quality control

303

Fig.3

```
┌────────────────────────────────────────────────────────────────┐
│ breaking a genome DNA into DNA fragments having a predetermined │ 401
│ size by means of physical or chemical method, to obtain DNA fragments │
└────────────────────────────────────────────────────────────────┘
                              ▼
┌────────────────────────────────────────────────────────────────┐
│ subjecting the DNA fragments to end-repairing,                 │ 402
│ to obtain end-repaired DNA fragments with a phosphorylated end  │
└────────────────────────────────────────────────────────────────┘
                              ▼
┌────────────────────────────────────────────────────────────────┐
│ adding an "A" base to the end-repaired DNA fragments at 3'-end to │ 403
│ obtain DNA fragments with the "A" base at 3'-end               │
└────────────────────────────────────────────────────────────────┘
                              ▼
┌────────────────────────────────────────────────────────────────┐
│ ligating an Index Adaptor to the DNA fragments with the "A" base at 3'- │ 404
│ end, to obtain DNA fragments ligated with the Index Adaptor    │
└────────────────────────────────────────────────────────────────┘
                              ▼
┌────────────────────────────────────────────────────────────────┐
│ amplifying the DNA fragments ligated with the Index Adaptor using │ 405
│ a primer comprising a sequence specific to the Index Adaptor,  │
│ to obtain an amplification product                             │
└────────────────────────────────────────────────────────────────┘
                              ▼
┌────────────────────────────────────────────────────────────────┐
│ determining the amplification product as the DNA library,      │ 406
│ if the amplification product meets the requirement of quality control │
└────────────────────────────────────────────────────────────────┘
```

Fig.4

```
┌────────────────────────────────────────────────────────────────┐
│ filtering out first unqualified data in the sequencing data,   │ 501
│ to obtain first qualified sequencing data                      │
└────────────────────────────────────────────────────────────────┘
                              ▼
┌────────────────────────────────────────────────────────────────┐
│ aligning the first qualified sequencing to a reference genome sequence by short-sequence │
│ alignment software, and calculating a first correlation parameter of the sequencing depth value │ 502
│ of each of the plurality of DNA samples and a second correlation parameter of the sequencing │
│ depth value for a given sequence tagged site region among the plurality of DNA samples │
└────────────────────────────────────────────────────────────────┘
                              ▼
┌────────────────────────────────────────────────────────────────┐
│ filtering out second unqualified sequencing data based on the first calculated correlation │ 503
│ parameter, to obtain second qualified sequencing data          │
└────────────────────────────────────────────────────────────────┘
                              ▼
┌────────────────────────────────────────────────────────────────┐
│ filtering out third unqualified sequencing data based on the second calculated correlation │ 504
│ parameter, to obtain third qualified sequencing data of the given sequence tagged site region │
└────────────────────────────────────────────────────────────────┘
```

Fig.5

| | |
|---|---|
| obtaining a first sequencing data set by filtering out unqualified data of which contains more than a predetermined proportion of bases having a low-quality from the sequencing data | 601 |
| obtaining a second sequencing data set by filtering out unqualified data of which contains more than 10% undetermined bases from the first sequencing data set | 602 |
| obtaining a third sequencing data set by filtering out unqualified data of which contains a sequencing adaptor sequence from the second sequencing data set after being aligned to a sequencing adaptor sequence library | 603 |
| obtaining the first qualified sequencing data by filtering out unqualified data of which contains an exogenous sequence from the third sequencing data set after being aligned to all introduced exogenous sequences | 604 |

Fig.6

| | |
|---|---|
| obtaining a lower quartile Q1, an upper quartile Q3 and an interquartile IQR by Quartile function and ranking all sequencing depth values of the plurality of DNA samples in the first qualified sequencing data in an ascending order | 701 |
| filtering out unqualified sequencing data of which has a sequencing depth value beyond a range of from Q1-1.5IQR to Q3+1.5IQR, to obtain the second qualified sequencing data | 701 |

Fig.7

Fig.8

Fig.9

| obtaining a lower quartile Q1, an upper quartile Q3 and an interquartile IQR by Quartile function and ranking all sequencing depth values of the given region among different DNA samples in the second qualified sequencing data in an ascending order | 1001 |

| filtering out unqualified sequencing data of which a median of the sequencing depth values of the given region among different DNA samples is zero or greater than Q3+1.5IQR, to obtain the third qualified sequencing data | 1002 |

Fig.10

depth distribution of different DNA samples for a same region

Fig.11

```
┌─────────────────────────────────────────────────────────────────────┐
│ subjecting a sequencing depth value of the sequence tagged site region │  1201
│ to normalization, to obtain a normalized sequencing depth value        │
└─────────────────────────────────────────────────────────────────────┘
                              │
                              ▼
┌─────────────────────────────────────────────────────────────────────┐
│ detecting an outlier of the sequencing depth value of the sequence     │
│ tagged site region based on the normalized sequencing depth value     │  1202
│ using the mathematical statistics method, to obtain the result showing │
│ the presence or the absence of the micro-deletion in the sequence      │
│ tagged site region of the chromosome in each of the plurality of DNA   │
│ samples                                                                │
└─────────────────────────────────────────────────────────────────────┘
```

Fig.12

```
┌─────────────────────────────────────────────────────────────────────┐
│ calculating a mean value and a variance based on all normalized        │
│ sequencing depth values of each of the plurality of DNA samples for a  │
│ same region, and obtaining a normal distribution curve with all of     │  1301
│ non-outliers for the same region, based on the calculated mean value   │
│ and variance of all normalized depth values of each of the plurality   │
│ of DNA samples for the same region                                     │
└─────────────────────────────────────────────────────────────────────┘
                              │
                              ▼
┌─────────────────────────────────────────────────────────────────────┐
│ calculating a probability for each of the plurality of DNA samples in   │  1302
│ every region with a specific sequencing depth value, based on the       │
│ normal distribution curve                                              │
└─────────────────────────────────────────────────────────────────────┘
                              │
                              ▼
┌─────────────────────────────────────────────────────────────────────┐
│ determining a first probability cut-off value based on the probability  │  1303
│ for each of the plurality of DNA samples in a given region with the     │
│ specific sequencing depth value                                        │
└─────────────────────────────────────────────────────────────────────┘
                              │
                              ▼
┌─────────────────────────────────────────────────────────────────────┐
│ obtaining a result R1 showing the presence of the micro-deletion in the │  1304
│ given region, if the probability for one of the plurality of DNA samples │
│ in the given region with the specific sequencing depth value is smaller  │
│ than the first probability cut-off value                               │
└─────────────────────────────────────────────────────────────────────┘
```

Fig.13

subjecting the result R1 to experimental verification to obtain a verification result, and
determining a second probability cut-off value based on the verification result, wherein the second probability cut-off value is smaller than the first probability cut-off value

1401

obtaining a result R2 showing the presence of the micro-deletion in the given region, if the probability for one of the plurality of DNA samples in the given region with the specific depth value is smaller than the second probability cut-off value

1402

Fig.14

calculating a first ratio D/S between the normalized depth value of the plurality of DNA samples for a given region and a median of all sequencing depth values of each of the plurality of DNA samples, based on the normalized depth value of the sequence tagged site region of the plurality of the plurality of DNA samples

1501

calculating a first ratio D/R between the normalized depth value of the plurality of DNA samples for the given region and a median of the sequencing depth values of all regions, based on the normalized depth value of the sequence tagged site region of the plurality of DNA samples

1502

training a first ratio cut-off value with the first ratio D/S using ID3 algorithm, and training a second ratio cut-off value with the first ratio D/R using ID3 algorithm

1503

obtaining the result showing the absence of the micro-deletion in the given region of the plurality of DNA samples, if the first ratio D/S is greater than the first ratio cut-off value

1504

obtaining the result showing the absence of the micro-deletion in the given region of the plurality of DNA samples, if the first ratio D/S is smaller than the first ratio cut-off value and the first ratio D/R is greater than the second ratio cut-off value

1505

obtaining the result showing the presence of the micro-deletion in the given region of the plurality of DNA samples, if the first ratio D/S is smaller than the first ratio cut-off value and the first ratio D/R is smaller than the second ratio cut-off value

1506

Fig.15

| diary density | whether or not using a real favicon | whether or not an account being real |
|---|---|---|
| s | no | no |
| m | no | no |
| m | no | yes |
| s | yes | no |

Fig.16

Fig.17

Fig.18

calculating a mean value and a variance based on all normalized depth values of each of the plurality of DNA samples for a same region — 1901

obtaining a normal distribution curve with all of non-outliers for the same region, based on the calculated mean value and variance of all normalized depth values of each of the plurality of DNA samples for the same region — 1902

calculating a probability for each of the plurality of DNA samples in every region with a specific sequencing depth value, based on the normal distribution curve — 1903

determining a third probability cut-off value based on the probability for each of the plurality of DNA samples in a given region with the specific sequencing depth value, and obtaining a result R3 showing the presence of the micro-deletion in the given region, if the probability for one of the plurality of DNA samples in the given region with the specific sequencing depth value is smaller than the third probability cut-off value — 1904

calculating a second ratio D/S between the normalized depth value of the plurality of DNA samples for the given region and a median of all sequencing depth values of each of the plurality of DNA samples in the result R3 — 1905

calculating a second ratio D/R between the normalized depth value of the plurality of DNA samples for the given region and a median of the sequencing depth values of all regions in the result R3 — 1906

training a third ratio cut-off value with the second ratio D/S using ID3 algorithm, and training a fourth ratio cut-off value with the second ratio D/R using ID3 algorithm — 1907

obtaining the result showing the absence of the micro-deletion in the given region of the plurality of DNA samples, if the second ratio D/S is greater than the third ratio cut-off value — 1908

obtaining the result showing the absence of the micro-deletion in the given region of the plurality of DNA samples, if the second ratio D/S is smaller than the third ratio cut-off value and the second ratio D/R is greater than the fourth ratio cut-off value — 1909

obtaining the result showing the presence of the micro-deletion in the given region of the plurality of DNA samples, if the second ratio D/S is smaller than the third ratio cut-off value and the second ratio D/R in the result R3 is smaller than the fourth ratio cut-off value — 1910

Fig.19

Fig.20

Fig.21

Fig.22

Fig.23

Fig.24

2501

| 2501 | 2502 | 2503 | 2504 |
|---|---|---|---|
| a capture probe obtaining device | hybridizing device | sequencing data obtaining device | micro-deletion result obtaining device |

Fig.25

| 2601 | 2602 | 2603 | 2604 |
|---|---|---|---|
| mean value and variance obtaining module | normal distribution curve obtaining module | probability calculating module | determining module |

Fig.26

| 2701 | 2702 |
|---|---|
| probability cut-off value determining module | determining module |

Fig.27

| 2801 | 2802 | 2803 | 2804 |
|---|---|---|---|
| ratio D/S obtaining module | ratio D/R obtaining module | ratio cut-off value obtaining module | determining module |

2805

determining module

2806

determining module

Fig.28

Fig.29

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• KR 20090112235 A

**Non-patent literature cited in the description**

• **MITCHELL, TOM M.** Machine Learning. Mc-Graw-Hill, 1997
• Molecular Cloning: A Laboratory Manual. **J. SAM-BROOK et al.** Science Press
• NimbleGen Arrays User's Guide. Roche NimbleGen, Inc, 07 July 2009